(19) Europäisches Patentamt
European Patent Office
Office européen des brevets

(11) **EP 3 995 996 A1**

(12) **EUROPEAN PATENT APPLICATION**
published in accordance with Art. 153(4) EPC

(43) Date of publication:
**11.05.2022 Bulletin 2022/19**

(21) Application number: **20834262.6**

(22) Date of filing: **21.05.2020**

(51) International Patent Classification (IPC):
**G06N 3/04** (2006.01)　　　**G06N 20/00** (2019.01)

(52) Cooperative Patent Classification (CPC):
**G06N 3/04; G06N 20/00**

(86) International application number:
**PCT/JP2020/020026**

(87) International publication number:
**WO 2021/002111 (07.01.2021 Gazette 2021/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.07.2019 JP 2019124419**

(71) Applicant: FUJIFILM Corporation
**Tokyo 106-8620 (JP)**

(72) Inventors:
• **HASEGAWA, Masataka**
　**Minamiashigara-shi, Kanagawa 250-0193 (JP)**
• **NAKAMURA, Naoki**
　**Minamiashigara-shi, Kanagawa 250-0193 (JP)**

(74) Representative: **Parker, Andrew James**
**Meissner Bolte Patentanwälte**
**Rechtsanwälte Partnerschaft mbB**
**Widenmayerstraße 47**
**80538 München (DE)**

(54) **LEARNING DEVICE, LEARNING DEVICE OPERATION METHOD, LEARNING DEVICE OPERATION PROGRAM, AND OPERATION DEVICE**

(57)　There are provided a learning apparatus, an operation method of the learning apparatus, an operation program of the learning apparatus, and an operating apparatus capable of further improving accuracy of prediction of a quality of a product by a machine learning model in a case where learning is performed by inputting, as learning input data, multi-dimensional physical-property relevance data, which is derived from multi-dimensional physical-property data of the product, to the machine learning model. In the learning apparatus, a learning unit performs learning by inputting the learning input data to the machine learning model and outputs a temporary machine learning model, the learning input data including relevance data which is derived from physical-property data of the product and includes a plurality of items. An extraction unit extracts a high-contribution item from the plurality of items of the relevance data by using the temporary machine learning model, the high-contribution item being the item of which a contribution to improvement of accuracy of prediction of the quality satisfies a preset condition. The learning unit performs learning by selectively inputting the relevance data of the high-contribution item to a second machine learning model and outputs the second machine learning model as a learned model to be provided for actual operation.

FIG. 11

**Description**

**BACKGROUND OF THE INVENTION**

1. Field of the Invention

A technique of the present disclosure relates to a learning apparatus, an operation method of the learning apparatus, an operation program of the learning apparatus, and an operating apparatus.

2. Description of the Related Art

A quality of a product is predicted using a machine learning model. In order to improve accuracy of prediction, JP2018-018354A proposes a machine learning model that learns, as learning input data, physical-property relevance data derived from physical-property data representing a physical property of a product.

In JP2018-018354A, as a product, foods and drinks such as coffee beans are exemplified. Further, in JP2018-018354A, as physical-property data, spectrum data such as near infrared (NIR) spectroscopic analysis data, Fourier transform infrared (FT-IR) spectroscopic analysis data, or nuclear magnetic resonance (NMR) spectroscopic analysis data, or image data obtained by imaging a product using a camera is exemplified. As physical-property relevance data, numerical values obtained from spectrum data, for example, a slope, a periodicity, an amplitude, a peak height, and a peak width of a waveform of a spectrum are exemplified. Further, as physical-property relevance data, image data itself obtained by imaging a product using a camera is exemplified.

**SUMMARY OF THE INVENTION**

The physical-property data may be data representing one type of physical properties of a product by one parameter, such as a weight of a product, and may be data representing one type of physical properties of a product by a plurality of parameters, such as the spectrum data or the image data. More specifically, in a case where the physical-property data is spectrum data, a spectrum is a physical property of a product, and, for example, a wave number and an intensity correspond to a plurality of parameters. In a case where the physical-property data is image data, a color is a physical property of a product, and a red pixel value, a green pixel value, and a blue pixel value correspond to a plurality of parameters. In a case where a parameter is regarded as a dimension, it can be said that the physical-property data is multi-dimensional physical-property data. On the other hand, it can be said that the physical-property data representing one type of physical properties of a product by one parameter is one-dimensional physical-property data. Hereinafter, physical-property data representing one type of physical properties of a product by a plurality of parameters is referred to as multi-dimensional physical-property data. Further, physical-property relevance data derived from the multi-dimensional physical-property data is referred to as multi-dimensional physical-property relevance data.

The multi-dimensional physical-property relevance data includes lots of numerical values and image data. Thus, the multi-dimensional physical-property relevance data has relatively many items. In the many items of the multi-dimensional physical-property relevance data, there is an item that significantly contributes to improvement of accuracy of prediction of a machine learning model, and there is an item that hardly contributes to improvement of accuracy of prediction of a machine learning model. For this reason, in a case where learning is performed by inputting the multi-dimensional physical-property relevance data to the machine learning model as it is, a result of learning is not improved so much. As a result, there is a concern that accuracy of prediction of the machine learning model reaches a limit at a relatively low level.

An object of the technique of the present disclosure is to provide a learning apparatus, an operation method of the learning apparatus, an operation program of the learning apparatus, and an operating apparatus capable of further improving accuracy of prediction of a quality of a product by a machine learning model in a case where the multi-dimensional physical-property relevance data derived from multi-dimensional physical-property data of the product is input to the machine learning model as learning input data and learning is performed.

In order to achieve the objective, according to an aspect of the present disclosure, there is provided a learning apparatus including: a first acquisition unit that acquires learning input data to be input to a machine learning model for predicting a quality of a product, the learning input data including multi-dimensional physical-property relevance data which is derived from multi-dimensional physical-property data representing a physical property of the product and includes a plurality of items; a temporary learning unit that inputs the learning input data to the machine learning model, performs learning, and outputs a temporary machine learning model; an extraction unit that extracts a high-contribution item from the plurality of items of the multi-dimensional physical-property relevance data by using the temporary machine learning model, the high-contribution item being the item of which a contribution to improvement of accuracy of prediction of the quality satisfies a preset condition; and a main learning unit that selectively inputs the multi-dimensional physical-property relevance data of the high-contribution item to the machine learning model, performs learning, and outputs the machine learning model as a learned model to be provided for actual operation.

Preferably, the learning input data includes production condition data which is set in a production process of the product.

Preferably, the multi-dimensional physical-property data includes spectrum data which is detected by performing spectroscopic analysis on the product.

Preferably, the multi-dimensional physical-property relevance data is a representative value of an intensity derived for each of a plurality of intervals obtained by dividing the spectrum data.

Preferably, the multi-dimensional physical-property data includes image data obtained by imaging the product.

Preferably, the product is produced by using a flow synthesis method.

Preferably, the learning apparatus further includes a derivation unit that derives the multi-dimensional physical-property relevance data by applying at least a part of an autoencoder to the multi-dimensional physical-property data.

Preferably, the autoencoder is learned by inputting the multi-dimensional physical-property data of the product of which the quality is higher than a preset level, and the derivation unit inputs the multi-dimensional physical-property data to the autoencoder, outputs output data, and derives the multi-dimensional physical-property relevance data based on difference data between the multi-dimensional physical-property data which is input to the autoencoder and the output data.

Preferably, the derivation unit inputs the multi-dimensional physical-property data to the autoencoder, outputs feature data from an encoder network of the autoencoder, and derives the multi-dimensional physical-property relevance data based on the feature data.

Preferably, the multi-dimensional physical-property data includes image data of a spectrum which is represented by spectrum data detected by performing spectroscopic analysis on the product.

Preferably, the derivation unit derives the multi-dimensional physical-property relevance data for each of a plurality of intervals obtained by dividing the spectrum data.

According to another aspect of the present disclosure, there is provided an operating apparatus including: a second acquisition unit that acquires the learned model which is output from the main learning unit of the learning apparatus; a third acquisition unit that acquires multi-dimensional physical-property relevance data for prediction which is data of a product of which a quality is unknown; a processing unit that inputs the multi-dimensional physical-property relevance data for prediction which is data of the product of which the quality is unknown, which is acquired by the third acquisition unit, to the learned model acquired by the second acquisition unit, and predicts the quality; and an output control unit that controls outputting of a prediction result of the quality by the learned model.

According to still another aspect of the present disclosure, there is provided an operation method of a learning apparatus, the operation method including: a first acquisition step of acquiring learning input data to be input to a machine learning model for predicting a quality of a product, the learning input data including multi-dimensional physical-property relevance data which is derived from multi-dimensional physical-property data representing a physical property of the product and includes a plurality of items; a temporary learning step of inputting the learning input data to the machine learning model, performing learning, and outputting a temporary machine learning model; an extraction step of extracting a high-contribution item from the plurality of items of the multi-dimensional physical-property relevance data by using the temporary machine learning model, the high-contribution item being the item of which a contribution to improvement of accuracy of prediction of the quality satisfies a preset condition; and a main learning step of selectively inputting the multi-dimensional physical-property relevance data of the high-contribution item to the machine learning model, performing learning, and outputting the machine learning model as a learned model to be provided for actual operation.

According to still another aspect of the present disclosure, there is provided an operation program of a learning apparatus, the program causing a computer to function as: a first acquisition unit that acquires learning input data to be input to a machine learning model for predicting a quality of a product, the learning input data including multi-dimensional physical-property relevance data which is derived from multi-dimensional physical-property data representing a physical property of the product and includes a plurality of items; a temporary learning unit that inputs the learning input data to the machine learning model, performs learning, and outputs a temporary machine learning model; an extraction unit that extracts a high-contribution item from the plurality of items of the multi-dimensional physical-property relevance data by using the temporary machine learning model, the high-contribution item being the item of which a contribution to improvement of accuracy of prediction of the quality satisfies a preset condition; and a main learning unit that selectively inputs the multi-dimensional physical-property relevance data of the high-contribution item to the machine learning model, performs learning, and outputs the machine learning model as a learned model to be provided for actual operation.

According to the technique of the present disclosure, it is possible to provide a learning apparatus, an operation method of the learning apparatus, an operation program of the learning apparatus, and an operating apparatus capable of further improving accuracy of prediction of a quality of a product by a machine learning model in a case where the multi-dimensional physical-property relevance data derived from multi-dimensional physical-property data of the product is input to the machine learning model as learning input data and the learning is performed.

BRIEF DESCRIPTION OF THE DRAWINGS

Fig. 1 is a diagram illustrating a machine learning system, a flow reaction apparatus, a physical-property analysis apparatus, and a quality evaluation apparatus.

Fig. 2 is a diagram illustrating an outline of processing in the flow reaction apparatus, the physical-property analysis apparatus, and the quality evaluation apparatus.

Fig. 3 is a diagram illustrating an outline of processing in a learning apparatus and an operating apparatus.

Fig. 4 is a diagram illustrating that production condition data, physical-property data, relevance data, and quality data are associated with a common ID.

Fig. 5 is a diagram illustrating the flow reaction apparatus that includes a reaction section including a T-shaped junction portion.

Fig. 6 is a diagram illustrating the flow reaction apparatus that includes a reaction section including a cross-shaped junction portion.

Fig. 7 is a diagram illustrating production condition data.

Fig. 8 is a diagram illustrating spectrum data and a spectrum.

Fig. 9 is a diagram illustrating quality data.

Fig. 10 is a block diagram illustrating a computer including the learning apparatus and the operating apparatus.

Fig. 11 is a block diagram illustrating a processing unit of a CPU of the learning apparatus.

Fig. 12 is a diagram illustrating a plurality of intervals obtained by dividing spectrum data.

Fig. 13 is a diagram illustrating a state where a first derivation unit derives relevance data for each of the plurality of intervals obtained by dividing spectrum data.

Fig. 14 is a diagram illustrating details of a learning unit.

Fig. 15 is a diagram illustrating processing of a first processing unit in temporary learning.

Fig. 16 is a diagram illustrating details of an extraction unit.

Fig. 17 is a diagram illustrating processing of a second processing unit.

Fig. 18 is a diagram illustrating processing of a third processing unit.

Fig. 19 is a diagram illustrating processing of a calculation unit.

Fig. 20 is a diagram illustrating a specific example of processing of the calculation unit.

Fig. 21 is a diagram illustrating contribution information.

Fig. 22 is a diagram illustrating processing of a determination unit.

Fig. 23 is a diagram illustrating processing of the first processing unit in main learning.

Fig. 24 is a block diagram illustrating a processing unit of a CPU of the operating apparatus.

Fig. 25 is a diagram illustrating processing of a fourth processing unit.

Fig. 26 is a diagram illustrating a quality prediction display screen.

Fig. 27 is a flowchart illustrating a processing procedure of the learning apparatus.

Fig. 28 is a flowchart illustrating a processing procedure of temporary learning.

Fig. 29 is a flowchart illustrating a processing procedure for extracting high-contribution items.

Fig. 30 is a flowchart illustrating a processing procedure of main learning.

Fig. 31 is a flowchart illustrating a processing procedure of the operating apparatus.

Fig. 32 is a table illustrating a determination coefficient of a molecular weight dispersion and a determination coefficient of a molecular weight in Comparative Examples and Example.

Fig. 33 is a diagram illustrating image data.

Fig. 34 is a diagram illustrating a plurality of regions obtained by dividing image data.

Fig. 35 is a diagram illustrating a state where the first derivation unit derives relevance data for each of the plurality of regions obtained by dividing image data.

Fig. 36 is a diagram illustrating a state where relevance data is derived by applying an autoencoder to physical-property data.

Fig. 37 is a diagram illustrating the autoencoder.

Fig. 38 is a diagram explaining convolution processing.

Fig. 39 is a diagram illustrating an image feature map.

Fig. 40 is a diagram illustrating that the autoencoder is prepared for each of a plurality of intervals of spectrum data.

Fig. 41 is a diagram illustrating a state where learning is performed by inputting, as learning input image data, image data of a spectrum of a product of which a quality is higher than a preset level, to the autoencoder.

Fig. 42 is a diagram illustrating a state where difference data between the input image data and the output image data of the autoencoder is derived.

Fig. 43 is a diagram illustrating a state where the first derivation unit derives, as relevance data, an average value and a sum of the difference data for each of the plurality of intervals obtained by dividing spectrum data.

Fig. 44 is a diagram illustrating a state where relevance data is derived based on the image feature map.

**DESCRIPTION OF THE PREFERRED EMBODIMENTS**

[First Embodiment]

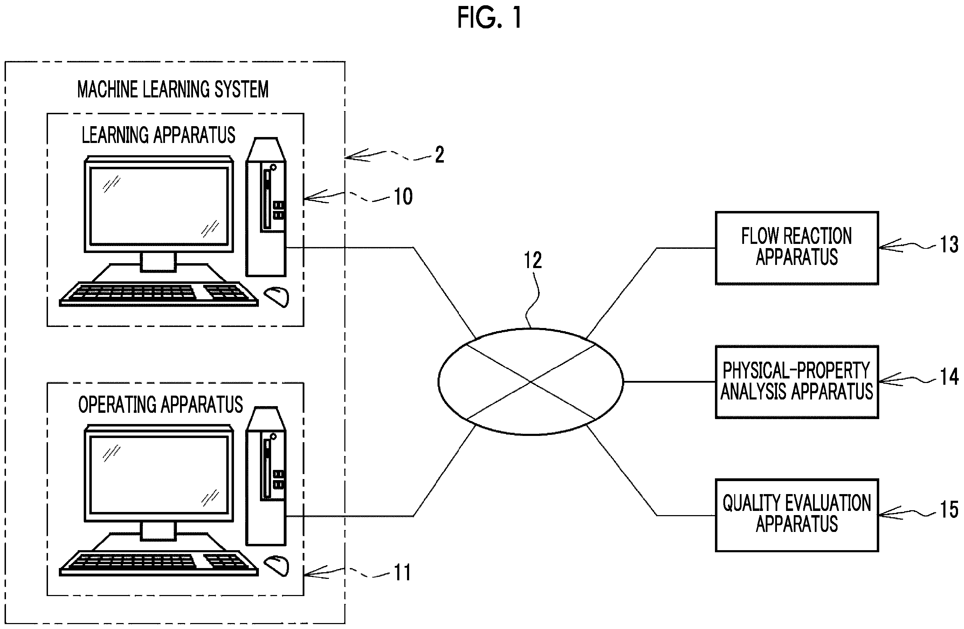

In Fig. 1, a machine learning system 2 includes a learning apparatus 10 and an operating apparatus 11. The learning apparatus 10 and the operating apparatus 11 are, for example, desktop personal computers. The learning apparatus 10 and the operating apparatus 11 are connected to each other so as to communicate with each other via a network 12. The network 12 is, for example, a local area network (LAN) or a wide area network (WAN) such as the Internet or a public communication network. A flow reaction apparatus 13, a physical-property analysis apparatus 14, and a quality evaluation apparatus 15 are also connected to the network 12.

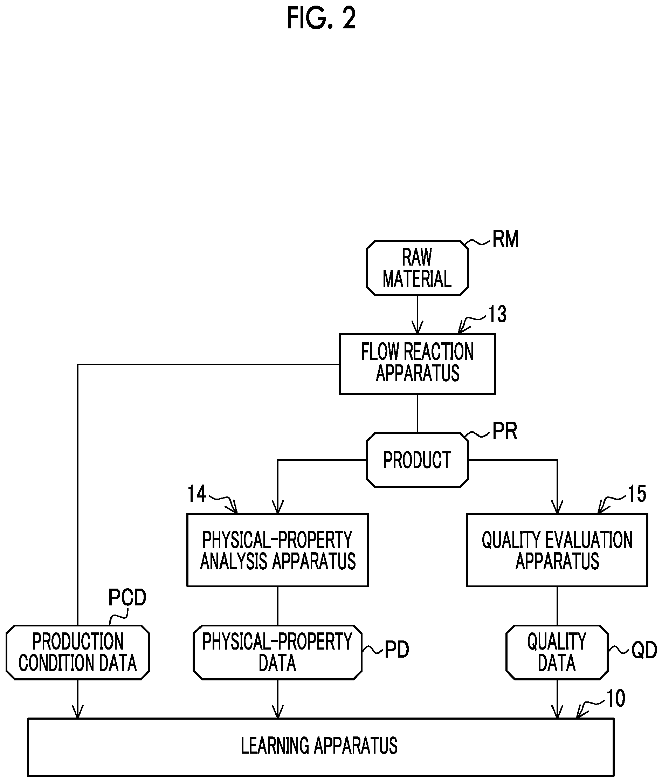

In Fig. 2, the flow reaction apparatus 13 produces a product PR from a raw material RM according to production condition data PCD of a production process by a flow synthesis method. The physical-property analysis apparatus 14 analyzes a physical-property of the product PR, and outputs physical-property data PD as an analysis result. The quality evaluation apparatus 15 evaluates a quality of the product PR, and outputs quality data QD as an evaluation result. The production condition data PCD from the flow reaction apparatus 13, the physical-property data PD from the physical-property analysis apparatus 14, and the quality data QD from the quality evaluation apparatus 15 are respectively transmitted to the learning apparatus 10.

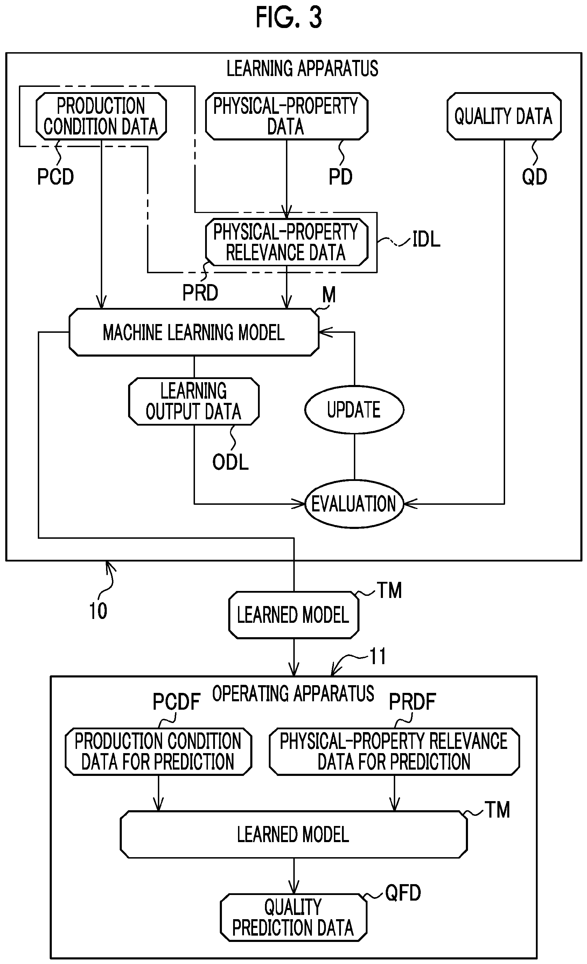

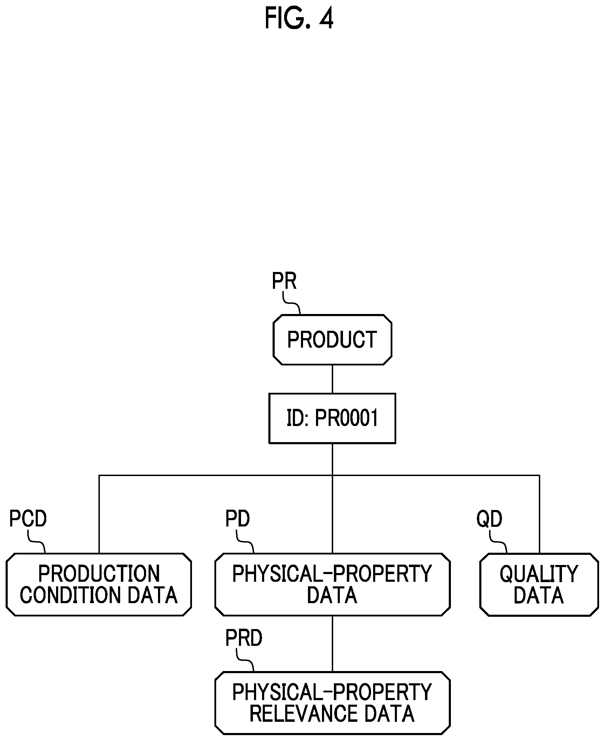

In Fig. 3, the learning apparatus 10 acquires the production condition data PCD from the flow reaction apparatus 13, the physical-property data PD from the physical-property analysis apparatus 14, and the quality data QD from the quality evaluation apparatus 15. The learning apparatus 10 derives physical-property relevance data (hereinafter, abbreviated as relevance data) PRD from the physical-property data PD. Learning input data IDL includes the relevance data PRD and the production condition data PCD. As illustrated in Fig. 4, the production condition data PCD, the physical-property data PD, the relevance data PRD derived from the physical-property data PD, and the quality data QD are associated with common identification data (ID) which is assigned to one product PR.

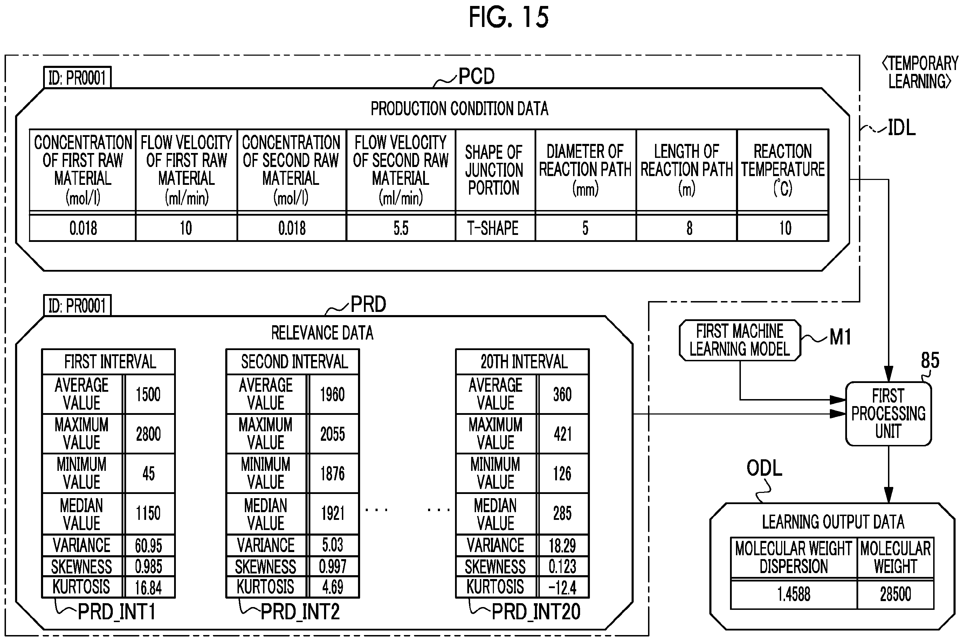

The learning apparatus 10 includes a machine learning model M. The machine learning model M is a model for predicting a quality of the product PR. In order to improve an accuracy of prediction of the machine learning model M, the learning apparatus 10 inputs the learning input data IDL including the production condition data PCD and the relevance data PRD with the same ID, to the machine learning model M (refer to Fig. 15). The machine learning model M outputs learning output data ODL according to the learning input data IDL.

The quality data QD is data for matching the learning output data ODL with an answer. As an accuracy of prediction of the machine learning model M is higher, a difference between the quality data QD and the learning output data ODL is smaller. Therefore, the learning apparatus 10 evaluates an accuracy of prediction of the machine learning model M by comparing the learning output data ODL with the quality data QD having the same ID as the learning input data IDL. The machine learning model M is updated according to the evaluation result. The learning apparatus 10 inputs the learning input data IDL to the machine learning model M, outputs the learning output data ODL from the machine learning model M, evaluates an accuracy of prediction of the machine learning model M, and updates the machine learning model M, while changing the learning input data IDL and the quality data QD. The series of processing is repeated until an accuracy of prediction of the machine learning model M reaches a preset level. The learning apparatus 10 transmits, to the operating apparatus 11, the machine learning model M of which an accuracy of prediction reaches a preset level, as a learned model TM to be used for actual operation.

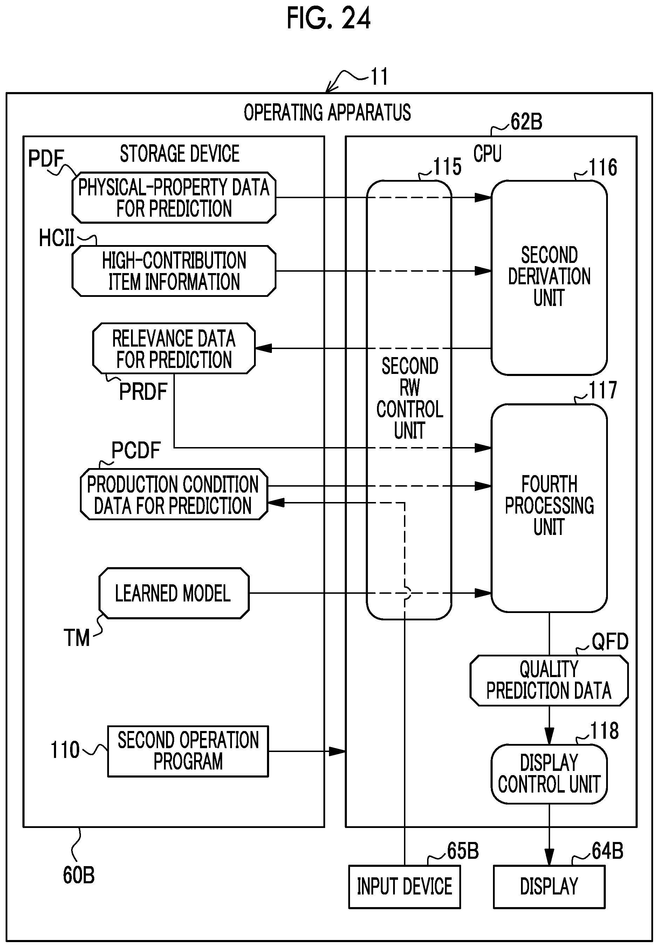

The operating apparatus 11 receives the learned model TM from the learning apparatus 10. The operating apparatus 11 inputs, to the learned model TM, production condition data for prediction PCDF, which is production condition data of a product PR of which a quality is unknown, and physical-property relevance data for prediction (hereinafter, abbreviated to as relevance data for prediction) PRDF, which is relevance data of the product PR of which a quality is unknown. The relevance data for prediction PRDF is data derived from the physical-property data for prediction PDF (refer to Fig. 24), which is physical-property data PD of the product PR of which a quality is unknown, similar to the relevance data PRD The learned model TM outputs quality prediction data QFD according to the production condition data for prediction PCDF and the relevance data for prediction PRDF.

A flow reaction performed in the flow reaction apparatus 13 is a synthesis reaction of synthesizing monomers, a polymerization reaction of generating a polymer by reacting monomers with each other, or the like. Therefore, the product PR may be, for example, a compound at a growth stage, which is a target of a polymerization reaction. In the example, the flow reaction apparatus 13 performs, as the flow reaction, an anionic polymerization reaction of polystyrene which is a product PR.

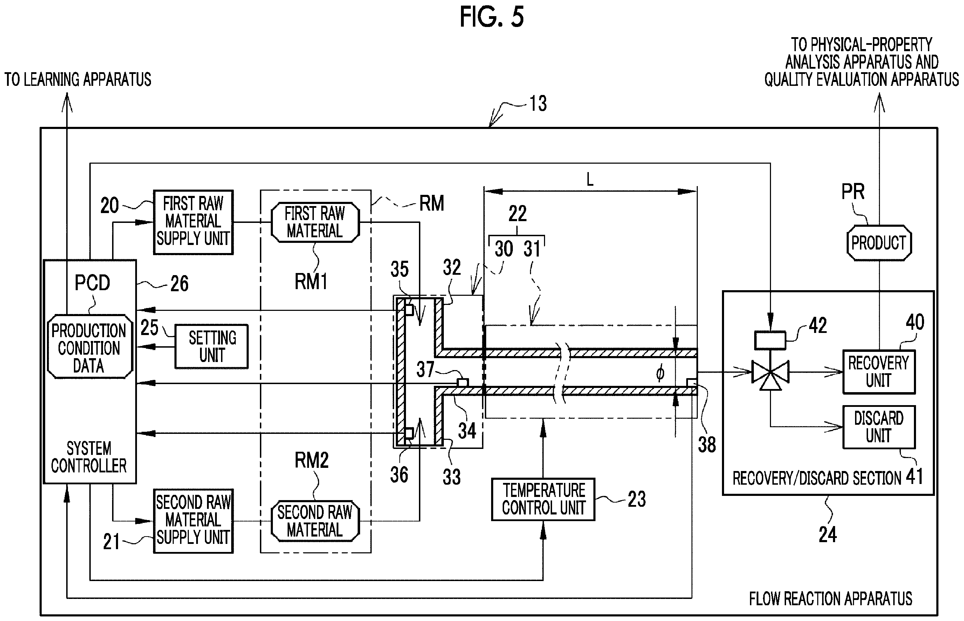

In Fig. 5, the flow reaction apparatus 13 includes a first raw material supply unit 20, a second raw material supply unit 21, a reaction section 22, a temperature control unit 23, a recovery/discard section 24, a setting unit 25, a system controller 26, and the like.

The first raw material supply unit 20 is connected to an upstream end of the reaction section 22 by a pipe (not illustrated). The first raw material supply unit 20 supplies a first raw material RM1 to the reaction section 22. The first raw material supply unit 20 includes a pump for transporting the first raw material RM1 to the reaction section 22. By controlling a rotation speed of the pump, a flow rate of the first raw material RM1 which is transported from the first raw material supply unit 20 to the reaction section 22 is adjusted.

In the example, the first raw material RM1 is a solution obtained by dissolving polystyryl lithium in a solvent. The polystyryl lithium functions as an initiator for the anionic polymerization reaction of polystyrene which is the product PR. As the solvent, tetrahydrofuran is used. In addition, a small amount of toluene and a small amount of hexane are mixed in the solution. A raw material for the flow reaction may be a mixture of a reactant such as polystyryl lithium and another substance, such as the first raw material RM1, or may be made of only a reactant.

Similar to the first raw material supply unit 20, the second raw material supply unit 21 is connected to the upstream end of the reaction section 22 by a pipe (not illustrated). The second raw material supply unit 21 supplies a second raw material RM2 to the reaction section 22. Similar to the first raw material supply unit 20, the second raw material supply unit 21 also includes a pump for transporting the second raw material RM2 to the reaction section 22. By controlling a rotation speed of the pump, a flow rate of the second raw material RM2 which is transported from the second raw material supply unit 21 to the reaction section 22 is adjusted.

In the example, the second raw material RM2 is an aqueous methanol solution. Methanol is used as a terminator for an anionic polymerization reaction.

The reaction section 22 is a section for performing a flow reaction (in the example, anionic polymerization reaction). The reaction section 22 includes a junction portion 30 and a reaction portion 31. The junction portion 30 includes a first pipe portion 32, a second pipe portion 33, and a third pipe portion 34. The first pipe portion 32 and the second pipe portion 33 are connected in a straight line, and the third pipe portion 34 intersects with the first pipe portion 32 and the second pipe portion 33 at a right angle. That is, the junction portion 30 has a T-shape.

The first pipe portion 32 is connected to the first raw material supply unit 20, and the second pipe portion 33 is connected to the second raw material supply unit 21. Further, the third pipe portion 34 is connected to the reaction portion 31. The first raw material RM1 is supplied from the first raw material supply unit 20 to the first pipe portion 32, and the second raw material RM2 is supplied from the second raw material supply unit 21 to the second pipe portion 33. The first raw material RM1 and the second raw material RM2 are mixed in the third pipe portion 34, and are transported to the reaction portion 31 in a mixed state.

A first flow velocity sensor 35 that detects a flow velocity of the first raw material RM1 passing through the first pipe portion 32 is provided in the first pipe portion 32. In addition, a second flow velocity sensor 36 that detects a flow velocity of the second raw material RM2 passing through the second pipe portion 33 is provided in the second pipe portion 33. Further, a third flow velocity sensor 37 that detects a flow velocity of the mixture of the first raw material RM1 and the second raw material RM2 passing through the third pipe portion 34 is provided in the third pipe portion 34.

The reaction portion 31 is an elongated pipe obtained by connecting a plurality of linear-shaped pipes having the same inner diameter in a straight line. A length L of the reaction portion 31 may be changed by changing the number of linear-shaped pipes to be connected and/or lengths of the linear-shaped pipes. Further, the inner diameter Ø of the reaction portion 31 may be changed by changing the inner diameter of the linear-shaped pipe to be connected.

The inside of the reaction portion 31 is a flow path through which the mixture of the first raw material RM1 and the second raw material RM2 flows, and is a portion at which a flow reaction is performed. In a case where the mixture passes through the reaction portion 31, a flow reaction is promoted, and thus a polystyrene solution is obtained. The flow reaction is slightly promoted in the third pipe portion 34 of the junction portion 30. On the other hand, a length of the third pipe portion 34 is very shorter than a length L of the reaction portion 31. For this reason, the length of the third pipe portion 34 is ignored, and the length L of the reaction portion 31 is regarded as a length of a reaction path, which is a length of a portion at which a flow reaction is performed. Similarly, the inner diameter Ø of the reaction portion 31 is regarded as a diameter of the reaction path, which is a diameter of a portion at which a flow reaction is performed.

The temperature control unit 23 includes a heater and/or a cooler, and controls a temperature inside the reaction portion 31 (hereinafter, referred to as a reaction temperature). A temperature sensor 38 for detecting the reaction temperature is provided at a downstream end of the reaction portion 31.

The recovery/discard section 24 is a section for recovering polystyrene which is the product PR and discarding a waste in which a reaction is failed. The recovery/discard section 24 includes a recovery unit 40 and a discard unit 41. The recovery unit 40 and the discard unit 41 are connected to the downstream end of the reaction portion 31 by a three-way valve 42. By using the three-way valve 42, switching between a recovery line that connects the reaction portion 31 and the recovery unit 40 and a discard line that connects the reaction portion 31 and the discard unit 41 can be performed.

The recovery unit 40 precipitates polystyrene from the polystyrene solution. The recovery unit 40 collects the precipitated polystyrene by filtering the solution. The collected polystyrene is dried. More specifically, the recovery unit 40 includes a container with a stirrer, and precipitates polystyrene by filling the container with methanol and mixing the polystyrene solution into the stirred methanol. Further, the recovery unit 40 includes a constant-temperature tank with a depressurization function, and dries the methanol by heating the inside of the constant-temperature tank in a depressurization state.

The discard unit 41 is a tank for storing a waste. Here, the waste is transported from the reaction portion 31 in a case where the flow velocity of the first raw material RM1, the flow velocity of the second raw material RM2, the flow velocity of the mixture, the reaction temperature, or the like is disturbed for some reason and, as a result, production cannot be performed under originally-predetermined production conditions.

The setting unit 25 receives setting of production conditions of the production process of the product PR by an operator of the flow reaction apparatus 13. The production conditions received by the setting unit 25 are registered in the system controller 26, as the production condition data PCD which is set in the production process of the product PR.

The system controller 26 overall controls operations of the entire flow reaction apparatus 13. The system controller 26 is connected to the first raw material supply unit 20, the second raw material supply unit 21, the temperature control unit 23, the first flow velocity sensor 35, the second flow velocity sensor 36, the third flow velocity sensor 37, the temperature sensor 38, and the three-way valve 42.

The system controller 26 adjusts the flow rate of the first raw material RM1 by controlling the rotation speed of the pump of the first raw material supply unit 20 according to the flow velocity of the first raw material RM1 that is detected by the first flow velocity sensor 35. Similarly, the system controller 26 adjusts the flow rate of the second raw material RM2 by controlling the rotation speed of the pump of the second raw material supply unit 21 according to the flow velocity of the second raw material RM2 that is detected by the second flow velocity sensor 36. In addition, the system controller 26 drives the temperature control unit 23 according to the reaction temperature detected by the temperature sensor 38. Further, the system controller 26 performs switching between the recovery line and the discard line by controlling the three-way valve 42.

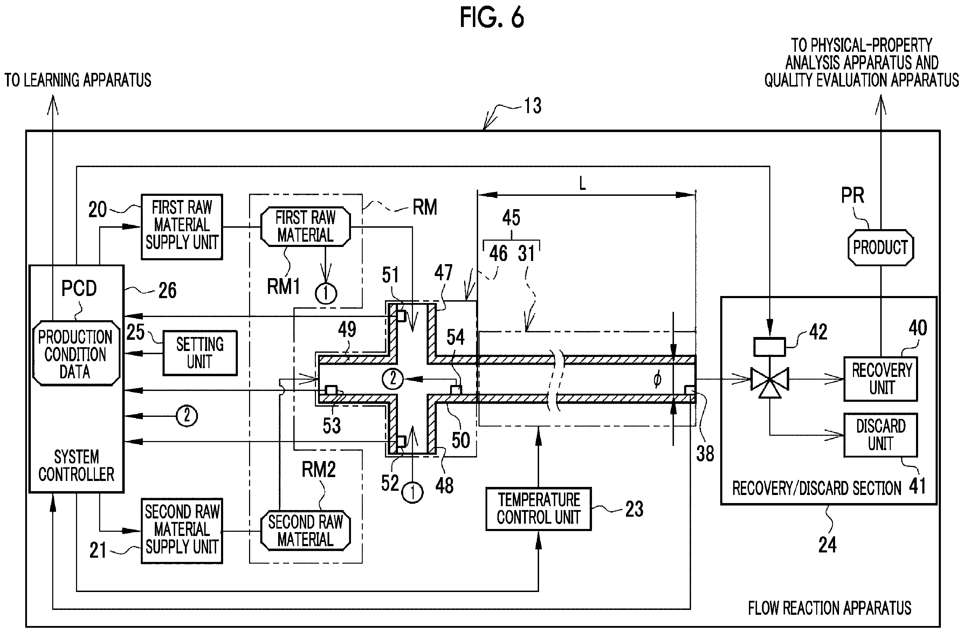

Instead of the reaction section 22, a reaction section 45 illustrated in Fig. 6 may be used. In Fig. 6, the same components as those in Fig. 5 are denoted by the same reference numerals, and a description thereof will be omitted.

The junction portion 46 of the reaction section 45 illustrated in Fig. 6 includes a first pipe portion 47, a second pipe portion 48, a third pipe portion 49, and a fourth pipe portion 50. The first pipe portion 47 and the second pipe portion 48 are connected in a straight line. Similarly, the third pipe portion 49 and the fourth pipe portion 50 are connected in a straight line. The first pipe portion 47 intersects with the third pipe portion 49 at a right angle, and the second pipe portion 48 intersects with the fourth pipe portion 50 at a right angle. That is, the junction portion 46 has a cross shape.

The first pipe portion 47 and the second pipe portion 48 are connected to the first raw material supply unit 20, and the third pipe portion 49 is connected to the second raw material supply unit 21. Further, the fourth pipe portion 50 is connected to the reaction portion 31. The first raw material RM1 is supplied from the first raw material supply unit 20 to the first pipe portion 47 and the second pipe portion 48, and the second raw material RM2 is supplied from the second raw material supply unit 21 to the third pipe portion 49. The first raw material RM1 and the second raw material RM2 are mixed in the fourth pipe portion 50, and are transported to the reaction portion 31 in a mixed state.

A first flow velocity sensor 51 and a second flow velocity sensor 52 that detect the flow velocity of the first raw material RM1 passing through the first pipe portion 47 and the second pipe portion 48 are provided in the first pipe portion 47 and the second pipe portion 48. In addition, a third flow velocity sensor 53 that detects the flow velocity of the second raw material RM2 passing through the third pipe portion 49 is provided in the third pipe portion 49. Further, a fourth flow velocity sensor 54 that detects the flow velocity of the mixture of the first raw material RM1 and the second raw material RM2 passing through the fourth pipe portion 50 is provided in the fourth pipe portion 50.

In this case, the system controller 26 adjusts the flow rate of the first raw material RM1 by controlling the rotation speed of the pump of the first raw material supply unit 20 according to an average value of the flow velocity of the first raw material RM1 that is detected by the first flow velocity sensor 51 and the flow velocity of the first raw material RM1 that is detected by the second flow velocity sensor 52. Further, the system controller 26 adjusts the flow rate of the second raw material RM2 by controlling the rotation speed of the pump of the second raw material supply unit 21 according to the flow velocity of the second raw material RM2 that is detected by the third flow velocity sensor 53.

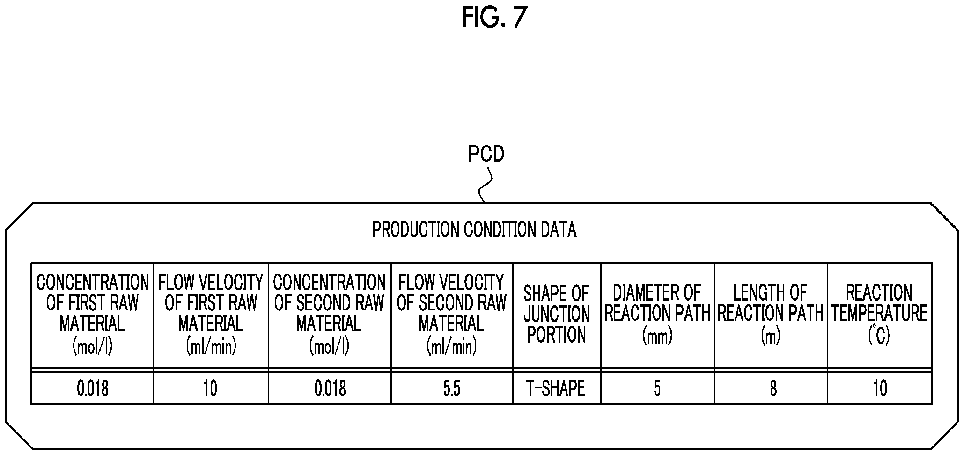

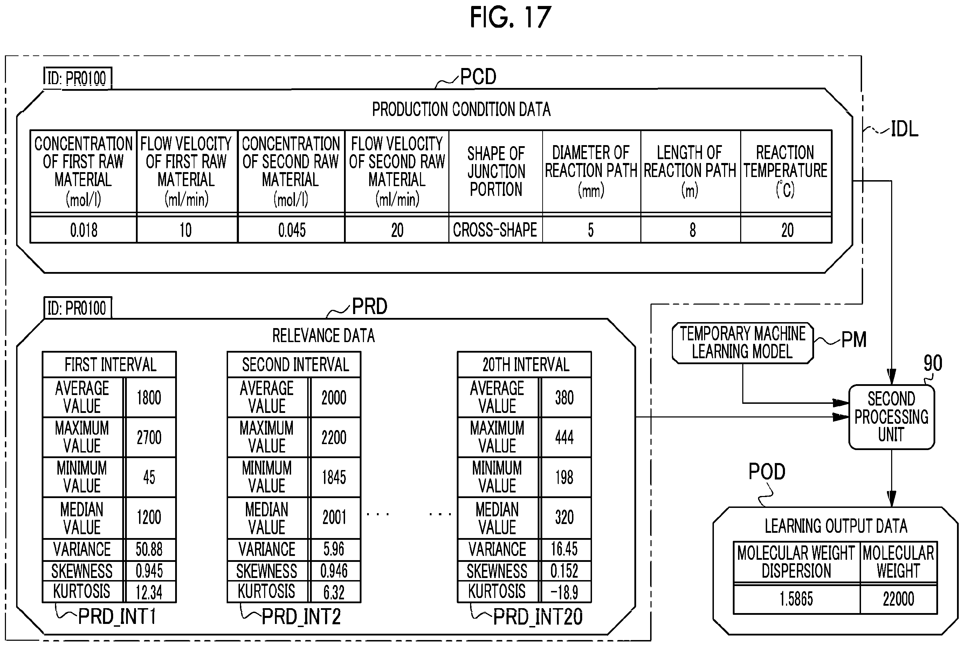

In Fig. 7, the production condition data PCD includes items of a concentration (unit: mol/l) and a flow velocity (unit: ml/min) of the first raw material RM1, a concentration (unit: mol/l) and a flow velocity (unit: ml/min) of the second raw material RM2, a shape of the junction portion, a diameter Ø (unit: mm) of the reaction path, a length L (unit: m) of the reaction path, and a reaction temperature (unit: °C). In the item of the shape of the junction portion, in a case where the reaction section 22 illustrated in Fig. 5 is used, "T-shape" illustrated in Fig. 7 is registered, and in a case where the reaction section 45 illustrated in Fig. 6 is used, "cross-shape" (refer to Fig. 17) is registered. The flow velocity of the mixture may be added as an item of the production condition data PCD.

In a case where the reaction section 22 is used, the system controller 26 adjusts the flow rate of the first raw material RM1 by controlling the rotation speed of the pump of the first raw material supply unit 20 such that the flow velocity of the first raw material RM1 detected by the first flow velocity sensor 35 matches with the flow velocity of the first raw material RM1 registered in the production condition data PCD. Similarly, the system controller 26 adjusts the flow rate of the second raw material RM2 by controlling the rotation speed of the pump of the second raw material supply unit 21 such that the flow velocity of the second raw material RM2 detected by the second flow velocity sensor 36 matches with the flow velocity of the second raw material RM2 registered in the production condition data PCD.

In a case where the reaction section 45 is used, the system controller 26 adjusts the flow rate of the first raw material RM1 by controlling the rotation speed of the pump of the first raw material supply unit 20 such that an average value of the flow velocity of the first raw material RM1 detected by the first flow velocity sensor 51 and the flow velocity of the first raw material RM1 detected by the second flow velocity sensor 52 matches with the flow velocity of the first raw material RM1 registered in the production condition data PCD. Similarly, the system controller 26 adjusts the flow rate of the second raw material RM2 by controlling the rotation speed of the pump of the second raw material supply unit 21 such that the flow velocity of the second raw material RM2 detected by the third flow velocity sensor 53 matches with the flow velocity of the second raw material RM2 registered in the production condition data PCD.

Further, the system controller 26 drives the temperature control unit 23 such that the reaction temperature detected by the temperature sensor 38 matches with a reaction temperature registered in the production condition data PCD.

In a case where a deviation between each value detected by each of the sensors 35, 36, 38, 51, 52, and 53 and each value registered in the production condition data PCD exceeds a preset range, the system controller 26 controls the three-way valve 42 to perform switching to the discard line and guide the waste to the discard unit 41. In a case where the reaction fails and the waste is generated, of course, the physical-property data PD and the quality data QD are not output. Therefore, in a case where the waste is generated, the production condition data PCD is discarded without being transmitted to the learning apparatus 10.

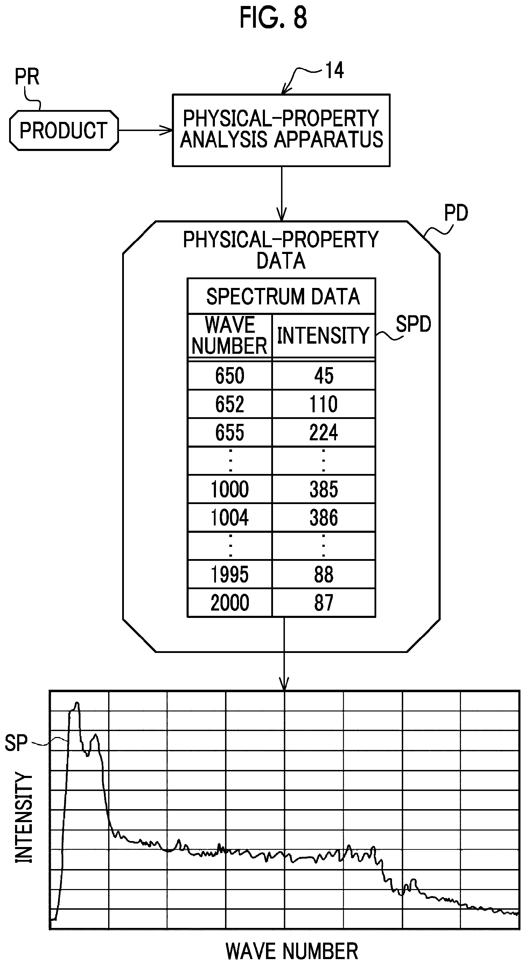

In Fig. 8, the physical-property analysis apparatus 14 detects, as the physical-property data PD, spectrum data SPD by performing spectroscopic analysis on the product PR. The physical-property analysis apparatus 14 performs, as spectroscopic analysis, for example, near-infrared spectroscopic analysis, Fourier transform infrared spectroscopic analysis, Raman spectroscopic analysis, and nuclear magnetic resonance spectroscopic analysis. The spectrum data SPD illustrated in Fig. 8 is obtained by Raman spectroscopic analysis, and includes a set of a wave number and an intensity. That is, the spectrum data SPD is an example of "multi-dimensional physical-property data" according to the technique of the present disclosure. A spectrum SP is obtained by plotting the intensity of the spectrum data SPD for each wave number and connecting the intensities with a line. The spectrum data SPD may include a set of a wavelength and an intensity instead of the set of the wave number and the intensity.

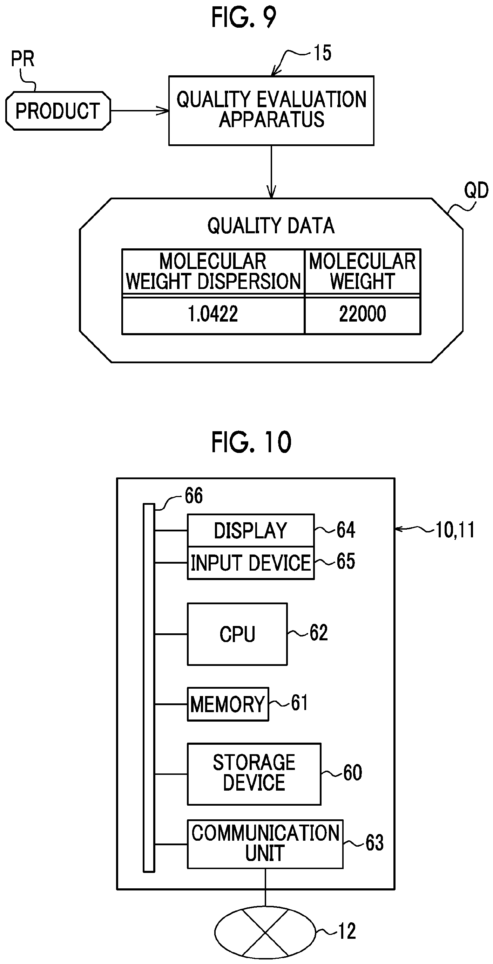

In Fig. 9, the quality evaluation apparatus 15 outputs, as the quality data QD, a molecular weight dispersion and a molecular weight of the product PR. Here, the molecular weight is a number average molecular weight. Further, the molecular weight dispersion is a value obtained by dividing a weight-average molecular weight by the number average molecular weight. The quality evaluation apparatus 15 obtains the molecular weight dispersion and the molecular weight by gel permeation chromatography (hereinafter, abbreviated as GPC) using, for example, a polystyrene solution obtained by dissolving polystyrene which is the product PR in tetrahydrofuran.

GPC is performed under the following conditions.

Apparatus: HLC-8220GPC (manufactured by Tosoh Corporation)
Detector: Differential refractometer (refractive index (RI) detector)
Pre-column: TSKGUARDCOLUMN HXL-L 6 mm $\times$ 40 mm (manufactured by Tosoh Corporation)
Sample-side column: Following three columns (1) to (3) are directly connected in order (all manufactured by Tosoh Corporation)

(1) TSK-GEL GMHXL 7.8 mm $\times$ 300 mm
(2) TSK-GEL G4000HXL 7.8 mm $\times$ 300 mm
(3) TSK-GEL G2000HXL 7.8 mm $\times$ 300 mm

Reference-side column: TSK-GEL G1000HXL 7.8 mm $\times$ 300 mm
Temperature of constant-temperature tank: 40°C
Movement layer: tetrahydrofuran
Flow rate of sample-side movement layer: 1.0 ml/min
Flow rate of reference-side movement layer: 1.0 ml/min
Concentration of sample : 0.1% by mass
Injection amount of sample: 100 $\mu$l
Collection time of data: 5 to 45 minutes after injection of sample
Sampling pitch: 300 msec

Instead of GPC, various methods such as infrared spectroscopic analysis, nuclear magnetic resonance spectroscopic analysis, high performance liquid chromatography (HPLC), or gas chromatography (GC) may be used. Further, the quality data QD is not limited to the molecular weight dispersion and the molecular weight of the product PR. In a case where the product PR is obtained in a state of a solution, a molar concentration which is a concentration of the product PR in the solution may be used as the quality data QD. Alternatively, a yield of the product PR that is obtained by dividing an amount of the product PR by an amount of the raw material RM may be used as the quality data QD. Further, in a case where a by-product is produced, a yield of the by-product may be used as the quality data QD.

In Fig. 10, the computer including the learning apparatus 10 and the computer including the operating apparatus 11 have the same basic configuration, and each of the computers includes a storage device 60, a memory 61, a central processing unit (CPU) 62, a communication unit 63, a display 64, and an input device 65. The components are connected to each other via a bus line 66.

The storage device 60 is a hard disk drive that is built in the computer including the learning apparatus 10 or the like or is connected via a cable or a network. Alternatively, the storage device 60 is a disk array in which a plurality of hard disk drives are connected in series. The storage device 60 stores a control program such as an operating system, various application programs, and various data associated with the programs. A solid state drive may be used instead of or in addition to the hard disk drive.

The memory 61 is a work memory which is necessary to execute processing by the CPU 62. The CPU 62 loads the program stored in the storage device 60 into the memory 61, and collectively controls each unit of the computer by executing processing according to the program.

The communication unit 63 is a network interface that controls transmission of various information via the network 12. The display 64 displays various screens. The computer constituting the learning apparatus 10 or the like receives an input of an operation instruction from the input device 65 via the various screens. The input device 65 includes a keyboard, a mouse, a touch panel, and the like.

In the following description, in order to distinguish the components, a subscript "A" is attached to each component of the learning apparatus 10, and a subscript "B" is attached to each component of the operating apparatus 11.

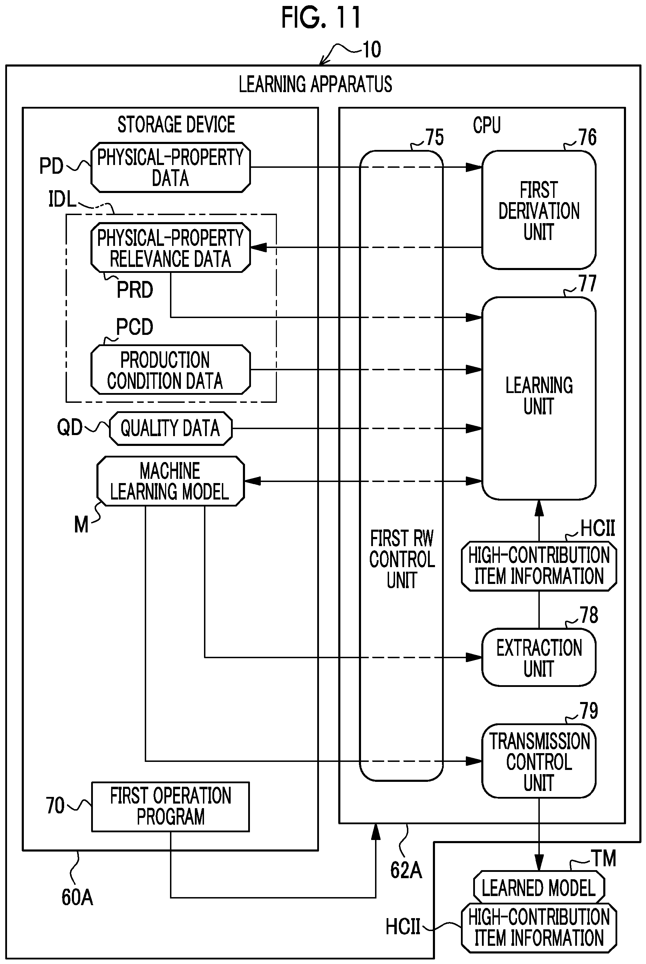

In Fig. 11, a first operation program 70 is stored in the storage device 60A of the learning apparatus 10. The first operation program 70 is an application program for causing the computer to function as the learning apparatus 10. That is, the first operation program 70 is an example of "the operation program of the learning apparatus" according to the technique of the present disclosure.

The storage device 60A also stores the production condition data PCD from the flow reaction apparatus 13, the physical-property data PD from the physical-property analysis apparatus 14, and the quality data QD from the quality evaluation apparatus 15. Further, the storage device 60A also stores the relevance data PRD derived from the physical-property data PD and the machine learning model M. A plurality of sets of the production condition data PCD, the physical-property data PD, the relevance data PRD, and the quality data QD are stored in the storage device 60A.

In a case where the first operation program 70 is started, the CPU 62A of the computer including the learning apparatus 10 functions as a first read/write (hereinafter, abbreviated as RW) control unit 75, a first derivation unit 76, a learning unit 77, an extraction unit 78, and a transmission control unit 79, in cooperation with the memory 61 and the like.

The first RW control unit 75 controls reading of various data stored in the storage device 60A and storing of various data in the storage device 60A. The first RW control unit 75 reads the physical-property data PD from the storage device 60A, and outputs the physical-property data PD to the first derivation unit 76. Further, the first RW control unit 75 stores the relevance data PRD from the first derivation unit 76 in the storage device 60A.

The first RW control unit 75 reads the relevance data PRD, the production condition data PCD, and the quality data QD from the storage device 60A, and outputs the read data to the learning unit 77. Since the relevance data PRD and the production condition data PCD are included in the learning input data IDL, the first RW control unit 75 acquires the learning input data IDL by reading the relevance data PRD and the production condition data PCD from the storage device 60A. That is, the first RW control unit 75 is an example of a "first acquisition unit" according to the technique of the present disclosure.

The first RW control unit 75 reads the machine learning model M from the storage device 60A, and outputs the machine learning model M to any of the learning unit 77, the extraction unit 78, and the transmission control unit 79. Further, the first RW control unit 75 stores the machine learning model M from the learning unit 77 in the storage device 60A.

The first derivation unit 76 receives the physical-property data PD from the first RW control unit 75. The first derivation unit 76 derives the relevance data PRD from the physical-property data PD. The first derivation unit 76 assigns the same ID as the ID of the physical-property data PD to the derived relevance data PRD, and outputs the relevance data PRD to the first RW control unit 75. The first derivation unit 76 derives the relevance data PRD each time new physical-property data PD is transmitted from the physical-property analysis apparatus 14.

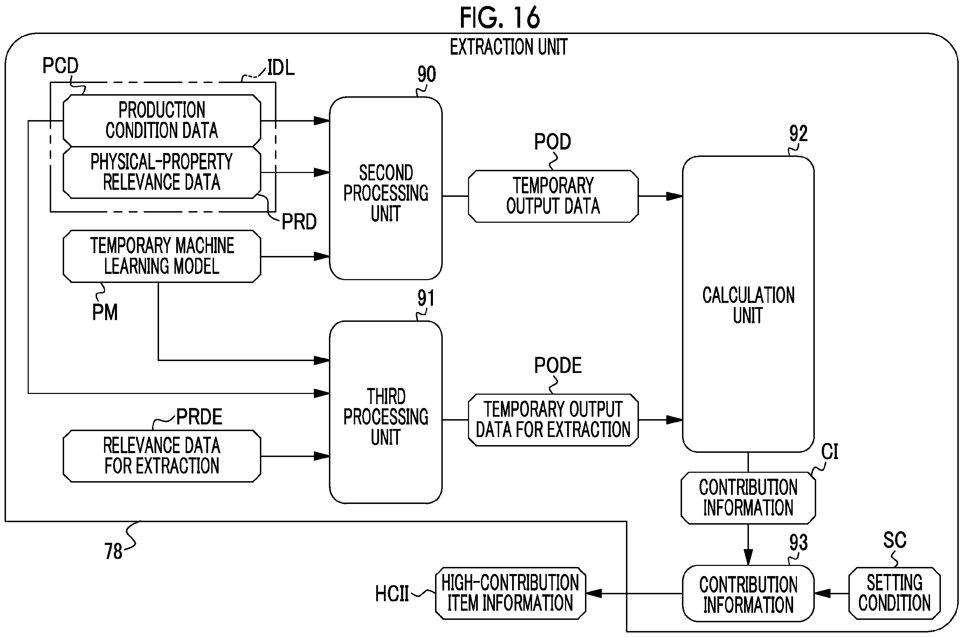

The learning unit 77 receives the learning input data IDL, the quality data QD, and the machine learning model M from the first RW control unit 75. The learning unit 77 performs learning by inputting the learning input data IDL to the machine learning model M, and outputs a temporary machine learning model PM (refer to Fig. 16). That is, the learning unit 77 is an example of a "temporary learning unit" according to the technique of the present disclosure. Hereinafter, the machine learning model M used during a period in which the learning unit 77 functions as the temporary learning unit is referred to as a first machine learning model M1 (refer to Fig. 15).

The extraction unit 78 receives the machine learning model M from the first RW control unit 75. The machine learning model M received by the extraction unit 78 from the first RW control unit 75 is a temporary machine learning model PM. The extraction unit 78 extracts a high-contribution item from a plurality of items of the relevance data PRD by using the temporary machine learning model PM. The high-contribution item is an item of which a contribution to improvement of accuracy of prediction of the quality of the product PR satisfies a preset condition. The extraction unit 78 outputs high-contribution item information HCII, which is an extraction result of the high-contribution item, to the learning unit 77. Although not illustrated, the extraction unit 78 outputs the high-contribution item information HCII to the first RW control unit 75, and the first RW control unit 75 stores the high-contribution item information HCII in the storage device 60A.

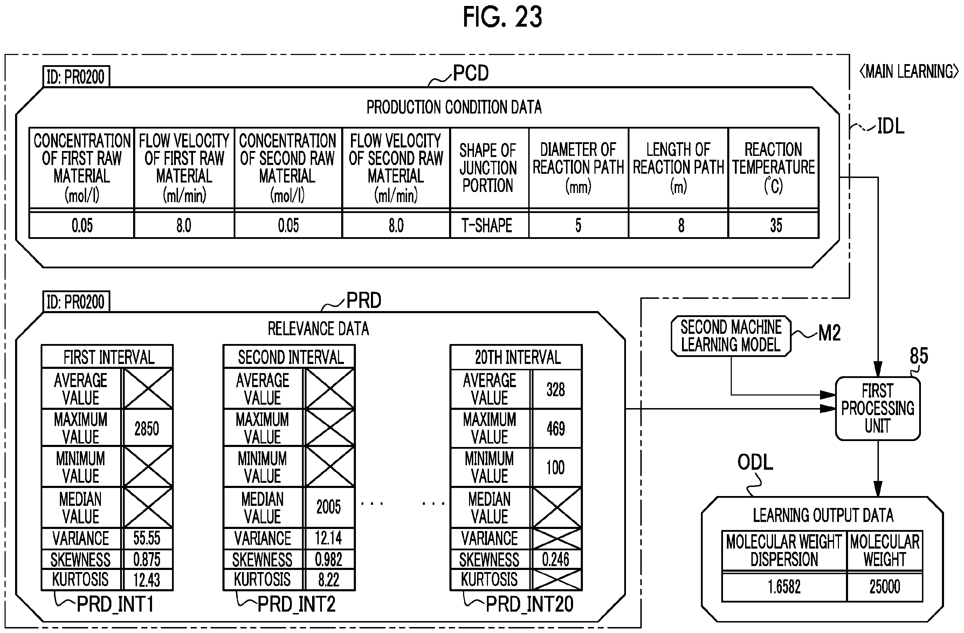

The learning unit 77 receives the high-contribution item information HCII from the extraction unit 78. The learning unit 77 performs learning by selectively inputting the relevance data PRD of the high-contribution item to the machine learning model M based on the high-contribution item information HCII, and outputs the machine learning model M as a learned model TM. That is, the learning unit 77 is an example of a "main learning unit" according to the technique of the present disclosure. Hereinafter, the machine learning model M used during a period in which the learning unit 77 functions as the main learning unit is referred to as a second machine learning model M2 (refer to Fig. 23).

The transmission control unit 79 receives the machine learning model M from the first RW control unit 75. The machine learning model M received by the transmission control unit 79 from the first RW control unit 75 is a learned model TM. The transmission control unit 79 performs a control for transmitting the learned model TM to the operating apparatus 11. Further, the transmission control unit 79 performs a control for receiving the learned model TM and the high-contribution item information HCII from the first RW control unit 75 and transmitting the high-contribution item information HCII to the operating apparatus 11.

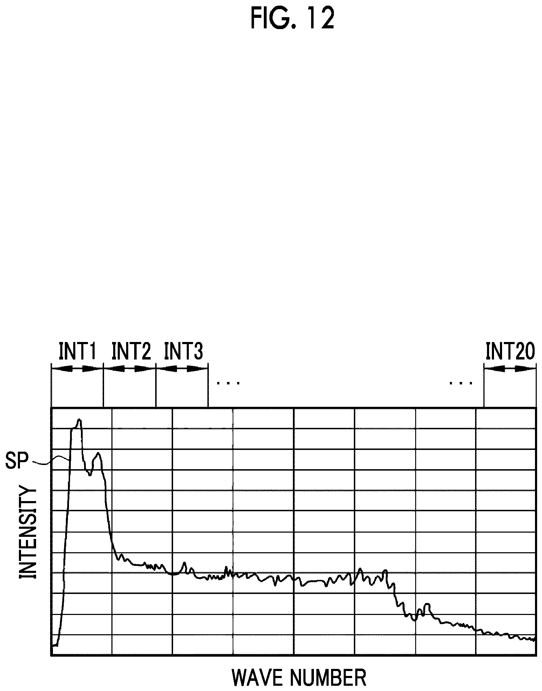

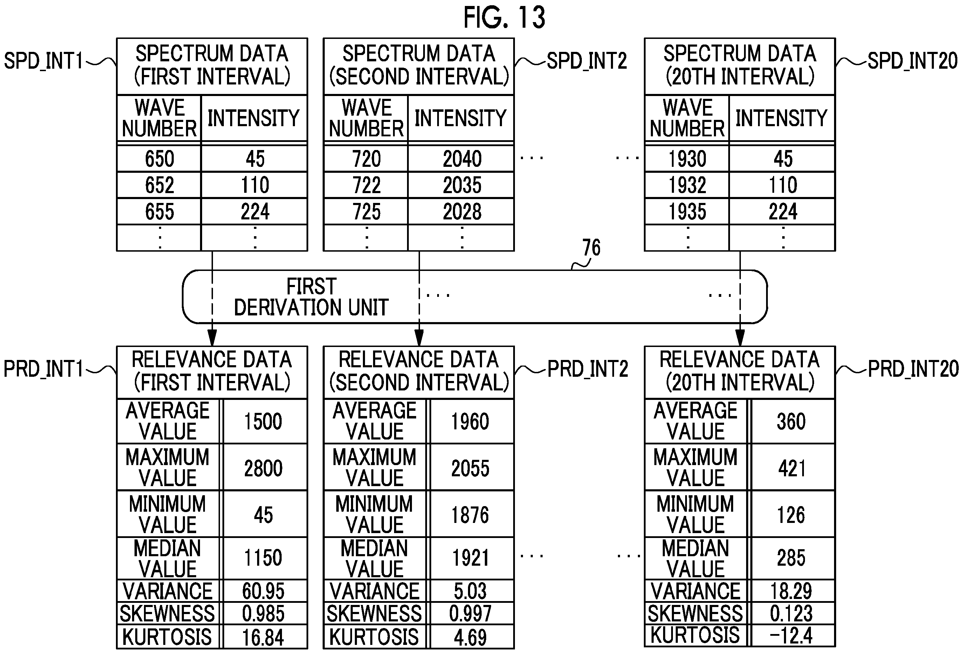

As illustrated in Fig. 12 and Fig. 13, the first derivation unit 76 derives the relevance data PRD for each of a plurality of intervals by which the spectrum data SPD is divided. The first derivation unit 76 derives, as the relevance data PRD, representative values of an intensity. The relevance data PRD is an example of "multi-dimensional physical-property relevance data" according to the technique of the present disclosure.

In Fig. 12, as the plurality of intervals by which the spectrum data SPD is divided, 20 intervals INT1, INT2, INT3, ⋯, and INT20 for dividing the spectrum data SPD into 20 equal parts are illustrated. Fig. 13 illustrates an example in which an average value, a maximum value, a minimum value, a median value, a variance, skewness, and kurtosis, which are representative values of intensities of pieces of the spectrum data SPD_INT1 to SPD_INT20 in the intervals INT1 to INT20, are derived as pieces of the relevance data PRD_INT1 to PRD_INT20. In a case of the example, the total number of items of the relevance data PRD obtained by integrating the pieces of the relevance data PRD_INT1 to PRD_INT20 is $7 \times 20 = 140$ because there are 7 types of the representative values and the intervals include 20 intervals.

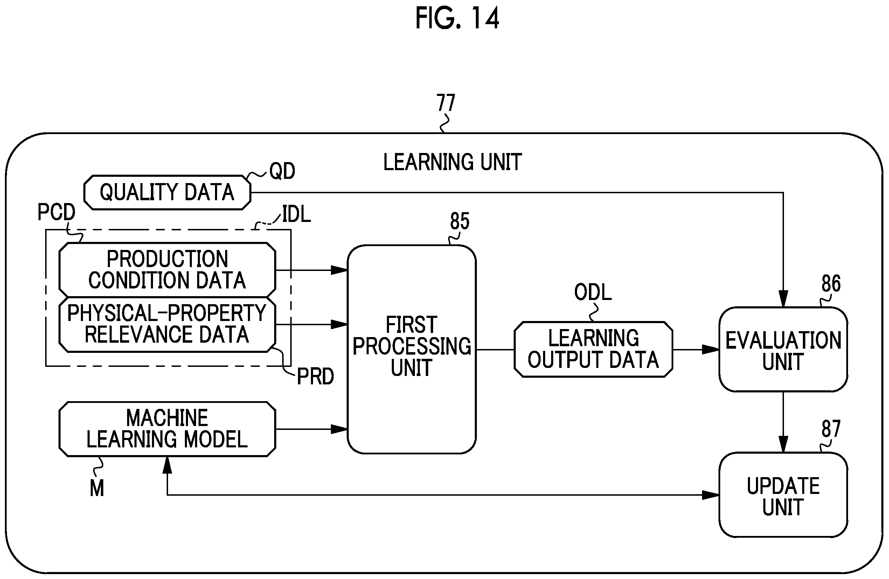

As illustrated in Fig. 14, the learning unit 77 includes a first processing unit 85, an evaluation unit 86, and an update unit 87. The first processing unit 85 outputs learning output data ODL from the machine learning model M by inputting the learning input data IDL to the machine learning model M. The learning output data ODL includes the molecular weight dispersion and the molecular weight, similar to the quality data QD (refer to Fig. 15 and the like). The first processing unit 85 outputs the learning output data ODL to the evaluation unit 86.

The evaluation unit 86 receives the learning output data ODL from the first processing unit 85. The evaluation unit 86 evaluates an accuracy of prediction of the machine learning model M by comparing the learning output data ODL and the quality data QD. The evaluation unit 86 outputs an evaluation result to the update unit 87.

The evaluation unit 86 evaluates an accuracy of prediction of the machine learning model M using, for example, a loss function. The loss function is a function that represents a degree of a difference between the learning output data ODL and the quality data QD. As a calculated value of the loss function is closer to 0, an accuracy of prediction of the machine learning model M is higher.

The update unit 87 updates the machine learning model M according to the evaluation result from the evaluation unit 86. For example, the update unit 87 changes various parameter values of the machine learning model M by a stochastic gradient descent method or the like using a learning coefficient. The learning coefficient indicates a change range in various parameter values of the machine learning model M. That is, as the learning coefficient has a relatively large value, the change range in various parameter values becomes wider, and thus, an update level of the machine learning model M becomes higher.

The inputting of the learning input data IDL to the machine learning model M and the outputting of the learning output data ODL to the evaluation unit 86 by the first processing unit 85, the evaluation of the accuracy of prediction by the evaluation unit 86, and the updating of the machine learning model M by the update unit 87 are repeated until the accuracy of prediction reaches a preset level.

As illustrated in Fig. 15, during a period in which the learning unit 77 functions as the temporary learning unit, the first processing unit 85 inputs, as the learning input data IDL, the production condition data PCD and the plurality of items of the relevance data PRD to the first machine learning model M1 without exception. The evaluation unit 86 evaluates an accuracy of prediction of the first machine learning model M1, and the update unit 87 updates the first machine learning model M1. By repeating the processing while changing the learning input data IDL, the accuracy of prediction of the first machine learning model M1 reaches a preset level. The first machine learning model M1 of which the accuracy of prediction reaches a preset level is stored in the storage device 60A by the first RW control unit 75, as the temporary machine learning model PM.

As illustrated in Fig. 16, the extraction unit 78 includes a second processing unit 90, a third processing unit 91, a calculation unit 92, and a determination unit 93. The second processing unit 90 outputs temporary output data POD from the temporary machine learning model PM by inputting the learning input data IDL to the temporary machine learning model PM. The temporary output data POD includes the molecular weight dispersion and the molecular weight, similar to the learning output data ODL (refer to Fig. 17 and the like). The second processing unit 90 outputs the temporary output data POD to the calculation unit 92.

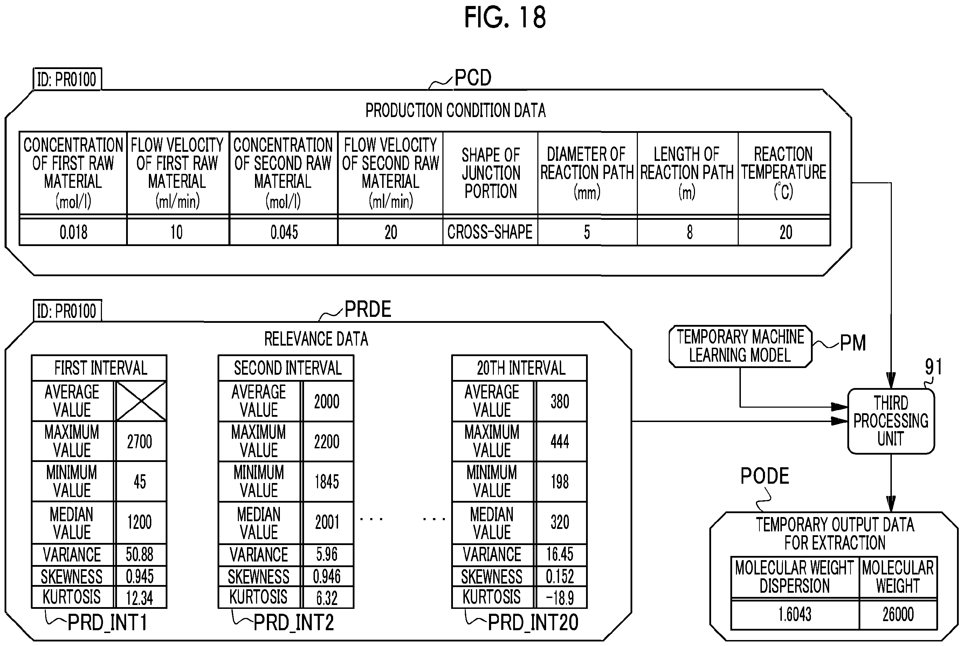

The third processing unit 91 outputs temporary output data for extraction PODE from the temporary machine learning model PM by inputting the production condition data PCD and physical-property relevance data for extraction (hereinafter, abbreviated as relevance data for extraction) PRDE to the temporary machine learning model PM, the production condition data PCD being same as the production condition data PCD which is input to the temporary machine learning model PM by the second processing unit 90. The temporary output data for extraction PODE includes the molecular weight dispersion and the molecular weight, similar to the learning output data ODL (refer to Fig. 18 and the like). The third processing unit 91 outputs the temporary output data for extraction PODE to the calculation unit 92.

The calculation unit 92 receives the temporary output data POD from the second processing unit 90 and the temporary output data for extraction PODE from the third processing unit 91. The calculation unit 92 calculates a contribution of the item of the relevance data PRD that is a degree to which the item of the relevance data PRD contributes to improvement of accuracy of quality prediction of the first machine learning model M1, based on the temporary output data POD and the temporary output data for extraction PODE. The calculation unit 92 outputs contribution information CI, which is a calculation result of the contribution, to the determination unit 93.

The determination unit 93 receives the contribution information CI from the calculation unit 92. The determination unit 93 determines whether or not each of the plurality of items of the relevance data PRD is a high-contribution item based on the contribution information CI and a setting condition SC. The determination unit 93 outputs, as a determination result, the high-contribution item information HCII to the learning unit 77.

As illustrated in Fig. 17, in the second processing unit 90, as in the case of the first processing unit 85 illustrated in Fig. 15, the plurality of items of the relevance data PRD are input to the temporary machine learning model PM without exception. On the other hand, as illustrated in Fig. 18, in the third processing unit 91, the relevance data for extraction PRDE, which is obtained by excluding one item among the plurality of items of the relevance data PRD, is input to the temporary machine learning model PM. Fig. 18 illustrates an example in which the item of the average value of the relevance data PRD_INT1 in the first interval INT1 is excluded.

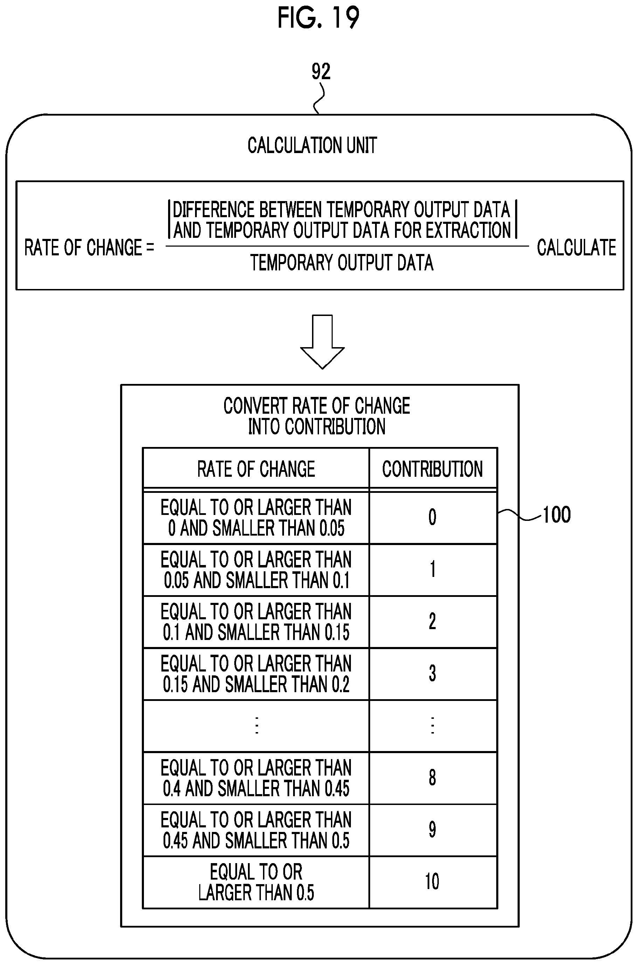

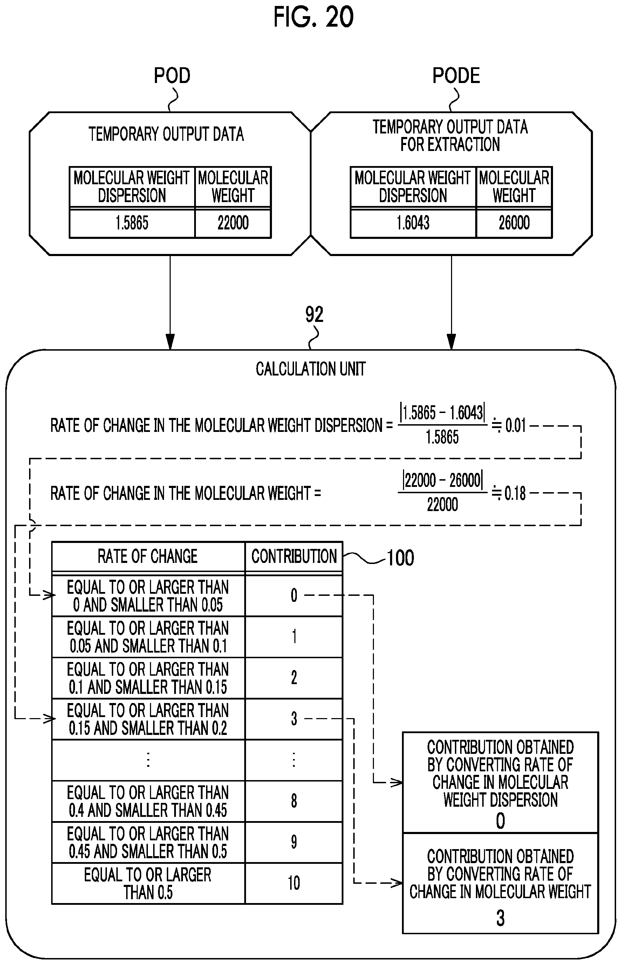

As illustrated in Fig. 19 and Fig. 20, first, the calculation unit 92 calculates a rate of change from the temporary output data POD and the temporary output data for extraction PODE. The rate of change is calculated by the following Equation 1.

$$\text{rate of change} = |\text{difference between temporary output data and temporary output data}$$

$$\text{for extraction}| / \text{temporary output data} \cdots \text{(Equation 1)}$$

The rate of change is a value indicating a degree to which the output data of the temporary machine learning model PM changes due to an influence of the item excluded from the relevance data for extraction PRDE.

Next, the calculation unit 92 converts the rate of change into a contribution by using a conversion table 100 that converts the rate of change into a contribution. In the conversion table 100, in a case where the rate of change is equal to or larger than 0 and smaller than 0.05, the contribution is registered as 0, in a case where the rate of change is equal to or larger than 0.05 and smaller than 0.1, the contribution is registered as 1, ···, in a case where the rate of change is equal to or larger than 0.45 and smaller than 0.5, the contribution is registered as 9, and in a case where the rate of change is equal to or larger than 0.5, the contribution is registered as 10.

Fig. 20 illustrates a case of the temporary output data POD illustrated in Fig. 17 that includes the molecular weight dispersion of 1.5865 and the molecular weight of 22000, and a case of the temporary output data for extraction PODE illustrated in Fig. 18 that includes the molecular weight dispersion of 1.6043 and the molecular weight of 26000. In this case, a rate of change in the molecular weight dispersion = |1.5865-1.6043|/1.5865≈0.01. According to the conversion table 100, in a case where the rate of change is 0.01, the contribution is 0. Thus, the contribution obtained by converting the rate of change in the molecular weight dispersion is calculated as 0. Similarly, a rate of change in the molecular weight = |22000-26000|/22000≈0.18, and thus, the contribution obtained by converting the rate of change in the molecular weight is calculated as 3. The contribution calculated in this way is a contribution of the item excluded in the relevance data for extraction PRDE (in Fig. 18, the item of the average value of the relevance data PRD_INT1).

The third processing unit 91 inputs the relevance data for extraction PRDE to the temporary machine learning model PM one after another while changing the excluded items one by one. In the example, since the number of the items of the relevance data PRD is 140, the third processing unit 91 inputs 140 pieces of the relevance data for extraction PRDE to the temporary machine learning model PM. In addition, the calculation unit 92 calculates the contribution for each relevance data for extraction PRDE. In order to improve reliability of a value of the rate of change, the rate of change may be calculated not only for one set of the production condition data PCD and the relevance data PRD but also for a plurality of different sets of the production condition data PCD and the relevance data PRD, and an average value of the rate of change may be converted to a contribution.

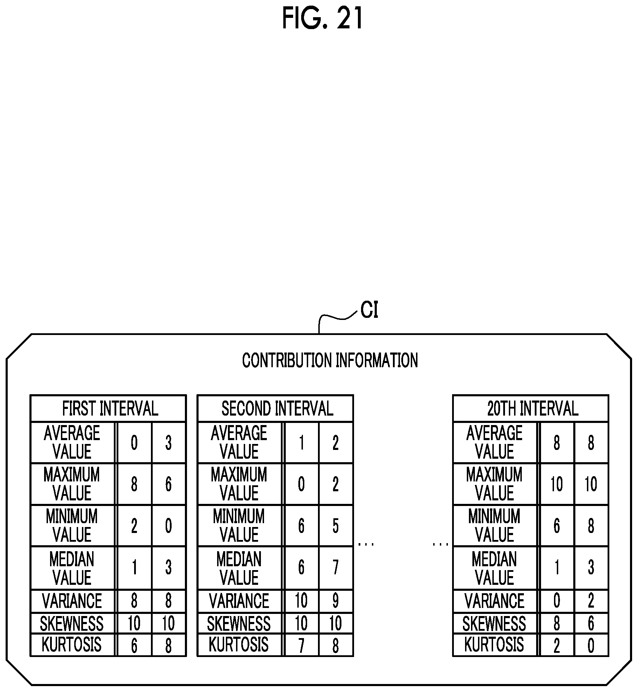

As illustrated in Fig. 21, in the contribution information CI, the contribution of each of the plurality of the items of the relevance data PRD is registered. The two numerical values next to each item are contributions, the left numerical value is the contribution obtained by converting the rate of change in the molecular weight dispersion, and the right numerical value is the contribution obtained by converting the rate of change in the molecular weight. For example, in the first interval INT1, the contribution obtained by converting the rate of change of the maximum value of the molecular weight dispersion is 8, and the contribution obtained by converting the rate of change in the molecular weight is 6.

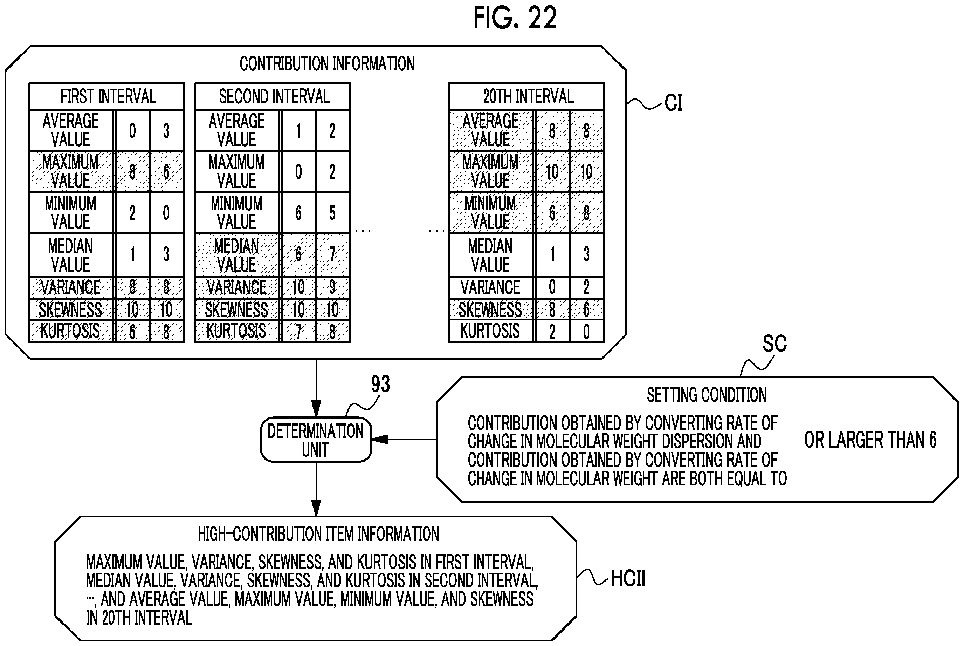

Fig. 22 illustrates, in the contribution information CI illustrated in Fig. 21, a case where the setting condition SC is "the contribution obtained by converting the rate of change in the molecular weight dispersion and the contribution obtained by converting the rate of change in the molecular weight are both equal to or larger than 6". In this case, the determination unit 93 determines that items, which are indicated by hatching and of which the contribution obtained by converting the rate of change in the molecular weight dispersion and the contribution obtained by converting the rate of change in the molecular weight are both equal to or larger than 6, are high-contribution items, the items including a maximum value, a variance, skewness, and kurtosis in the first interval INT1, a median value, a variance, skewness, and kurtosis in the second interval INT2, ···, and an average value, a maximum value, a minimum value, and skewness in the 20th interval INT20.

In this way, a method of extracting an essential part (in the example, the high-contribution item) from a plurality of pieces of data (in the example, the plurality of items of the relevance data PRD) is called sparse modeling. The sparse modeling may be performed, for example, using a glmnet package that can operate on a R language. A detail algorithm for sparse modeling is described in, for example, Regularization Paths for Generalized Linear Models via Coordinate Descent, Journal of statistical software, vol. 33-1 (2010)".

As illustrated in Fig. 23, during the period for which the learning unit 77 functions as the main learning unit, in the first processing unit 85, the relevance data PRD of the high-contribution item is selectively input to the second machine learning model M2. In other words, items other than the high-contribution items are selectively excluded, and the high-contribution items are input to the second machine learning model M2. The evaluation unit 86 evaluates an accuracy of prediction of the second machine learning model M2, and the update unit 87 updates the second machine learning model M2. By repeating the processing while changing the learning input data IDL, the accuracy of prediction of the second machine learning model M2 reaches a preset level. The second machine learning model M2 of which the accuracy of prediction reaches a preset level is stored in the storage device 60A by the first RW control unit 75, as the learned model TM. The second machine learning model M2 used during the period for which the learning unit 77 functions as the main learning unit is the same as the first machine learning model M1 used during the period for which the learning unit 77 functions as the temporary learning unit, in terms of characteristics such as a type and a performance.

In Fig. 24, a second operation program 110 is stored in the storage device 60B of the operating apparatus 11. The second operation program 110 is an application program for causing the computer to function as the operating apparatus 11.

The storage device 60B also stores the learned model TM and the high-contribution item information HCII from the learning apparatus 10 and the physical-property data for prediction PDF from the physical-property analysis apparatus 14. In addition, the storage device 60B also stores the production condition data for prediction PCDF. The production condition data for prediction PCDF is input via the input device 65B by the operator. More specifically, an input screen including input boxes for each item of the production condition data for prediction PCDF is displayed on the display 64B, and the production condition data for prediction PCDF is input via the input screen. The production condition data for prediction PCDF and the physical-property data for prediction PDF are the production condition data PCD and the physical-property data PD of the product PR of which a quality is unknown, and are used to predict the quality by using the learned model TM.

Further, the storage device 60B also stores the physical-property relevance data for prediction (hereinafter, abbreviated as relevance data for prediction) PRDF derived from the physical-property data for prediction PDF.

In a case where the second operation program 110 is started, the CPU 62B of the computer including the operating apparatus 11 functions as a second RW control unit 115, a second derivation unit 116, a fourth processing unit 117, and a display control unit 118 in cooperation with the memory 61 and the like.

Similar to the first RW control unit 75 of the learning apparatus 10, the second RW control unit 115 controls reading of various data stored in the storage device 60B and storing of various data in the storage device 60B. The second RW control unit 115 reads the physical-property data for prediction PDF and the high-contribution item information HCII from the storage device 60B, and outputs the physical-property data for prediction PDF and the high-contribution item information HCII to the second derivation unit 116. Further, the second RW control unit 115 stores the relevance data for prediction PRDF from the second derivation unit 116 in the storage device 60B.

The second RW control unit 115 reads the learned model TM from the storage device 60B, and outputs the learned model TM to the fourth processing unit 117. The second RW control unit 115 acquires the learned model TM by reading the learned model TM from the storage device 60B. That is, the second RW control unit 115 is an example of a "second acquisition unit" according to the technique of the present disclosure.

The second RW control unit 115 reads the relevance data for prediction PRDF and the production condition data for prediction PCDF from the storage device 60B, and outputs the read data to the fourth processing unit 117. The second RW control unit 115 acquires the relevance data for prediction PRDF by reading the relevance data for prediction PRDF from the storage device 60B. That is, the second RW control unit 115 is an example of a "third acquisition unit" according to the technique of the present disclosure.

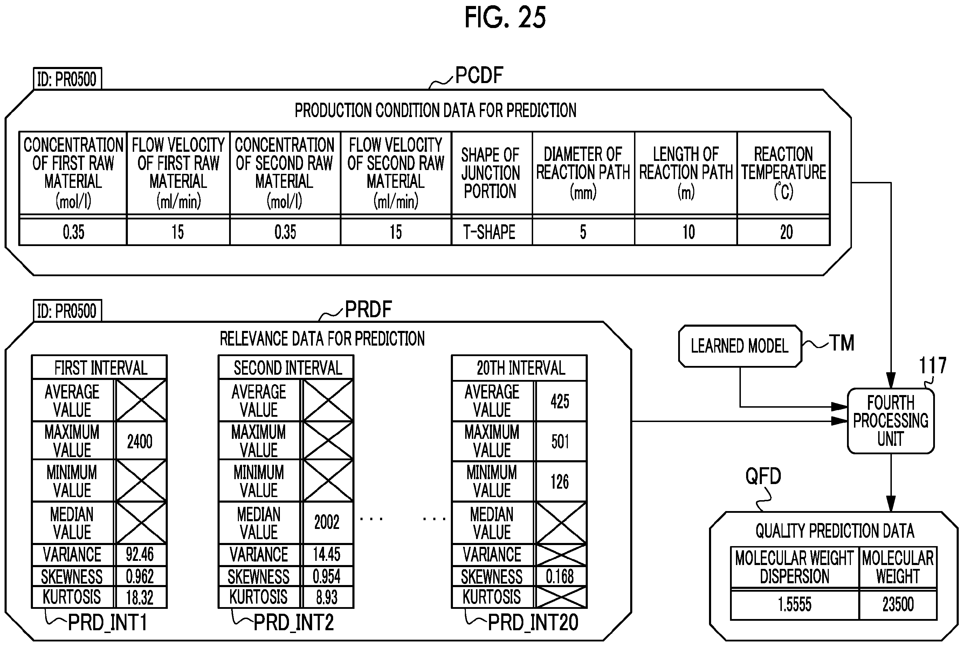

The second derivation unit 116 receives the physical-property data for prediction PDF and the high-contribution item information HCII from the second RW control unit 115. The second derivation unit 116 derives the relevance data for prediction PRDF from the physical-property data for prediction PDF. More specifically, similar to the first derivation unit 76 of the learning apparatus 10, the second derivation unit 116 derives an average value, a maximum value, a minimum value, a median value, a variance, skewness, and kurtosis of an intensity for each of the plurality of intervals INT1 to INT20 obtained by dividing the spectrum data SPD. Here, the second derivation unit 116 selectively derives the high-contribution item based on the high-contribution item information HCII, and does not derive items other than the high-contribution item. Therefore, similar to the relevance data PRD illustrated in Fig. 23, in the relevance data for prediction PRDF, items other than the high-contribution items are selectively excluded (refer to Fig. 25).

The fourth processing unit 117 receives the production condition data for prediction PCDF, the relevance data for prediction PRDF, and the learned model TM from the second RW control unit 115. The fourth processing unit 117 predicts a quality by inputting the production condition data for prediction PCDF and the relevance data for prediction PRDF to the learned model TM. That is, the fourth processing unit 117 is an example of a "processing unit" according to the technique of the present disclosure. The fourth processing unit 117 outputs quality prediction data QFD, which is a quality prediction result by the learned model TM, to the display control unit 118. The quality prediction data QFD includes the molecular weight dispersion and the molecular weight, similar to the quality data QD (refer to Fig. 25 and the like).

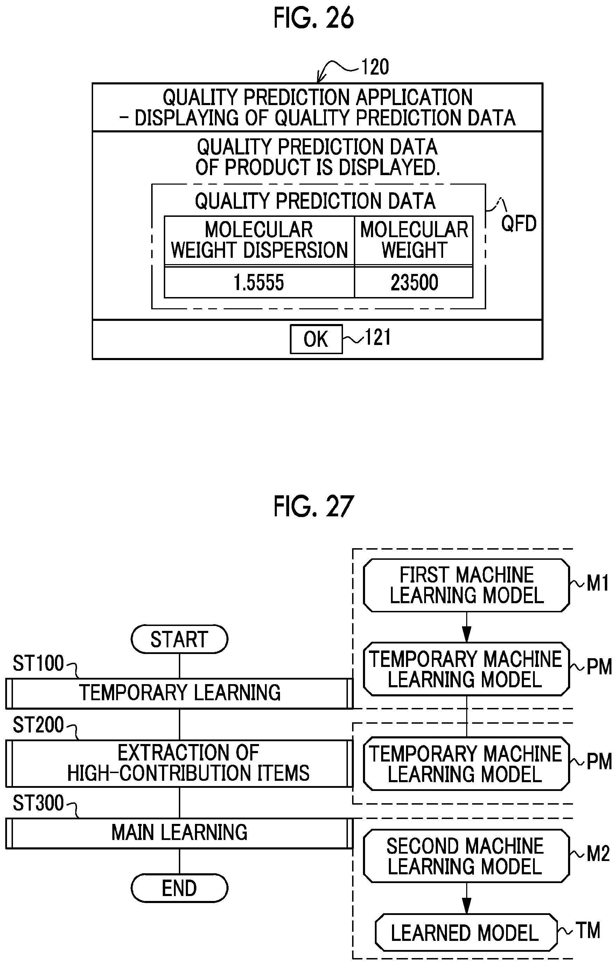

The display control unit 118 controls displaying of various screens on the display 64B. The various screens include a quality prediction display screen 120 (refer to Fig. 26) for displaying the quality prediction data QFD, in addition to the input screen for the production condition data for prediction PCDF. That is, the display control unit 118 is an example of an "output control unit" according to the technique of the present disclosure.

Fig. 25 illustrates a method of predicting the quality of the product PR of which an ID is PR0500 and of which the quality is unknown by using the learned model TM. Similar to the case illustrated in Fig. 23, in the fourth processing unit 117, the relevance data for prediction PRDF of the high-contribution item among the plurality of items is selectively input to the learned model TM. In other words, items other than the high-contribution items are selectively excluded, and the high-contribution items are input to the learned model TM.

In Fig. 26, on the quality prediction display screen 120 displayed on the display 64B under a control of the display control unit 118, the quality prediction data QFD which is output from the learned model TM is displayed. The display of the quality prediction display screen 120 disappears in a case where an OK button 121 is selected.

Next, an operation according to the configuration will be described with reference to flowcharts illustrated in Fig. 27 to Fig. 31. First, in a case where the first operation program 70 is started in the learning apparatus 10, as illustrated in Fig. 11, the CPU 62A of the learning apparatus 10 functions as the first RW control unit 75, the first derivation unit 76, the learning unit 77, the extraction unit 78, and the transmission control unit 79. As illustrated in Fig. 14, the learning unit 77 functions as the first processing unit 85, the evaluation unit 86, and the update unit 87. Further, as illustrated in Fig. 16, the extraction unit 78 functions as the second processing unit 90, the third processing unit 91, the calculation unit 92, and the determination unit 93.

In the learning apparatus 10, the first RW control unit 75 reads the spectrum data SPD, which is the physical-property data PD, from the storage device 60A, and outputs the spectrum data SPD to the first derivation unit 76. As illustrated in Fig. 12 and Fig. 13, the first derivation unit 76 derives, as the relevance data PRD, representative values (an average value, a maximum value, a minimum value, a median value, a variance, skewness, and kurtosis) of the intensity for each of the plurality of intervals INT1 to INT20 obtained by dividing the spectrum data SPD. The relevance data PRD is output from the first derivation unit 76 to the first RW control unit 75, and is stored in the storage device 60A by the first RW control unit 75. The derivation of the relevance data PRD is performed each time new physical-property data PD is transmitted from the physical-property analysis apparatus 14.

In Fig. 27, in the learning apparatus 10, temporary learning (step ST100), extraction of the high-contribution items (step ST200), and main learning (step ST300) are mainly performed. In the temporary learning, the first machine learning model M1 is learned, and is used as the temporary machine learning model PM. The temporary machine learning model PM is used to extract the high-contribution items. In the main learning, the second machine learning model M2 is learned, and is used as the learned model TM.

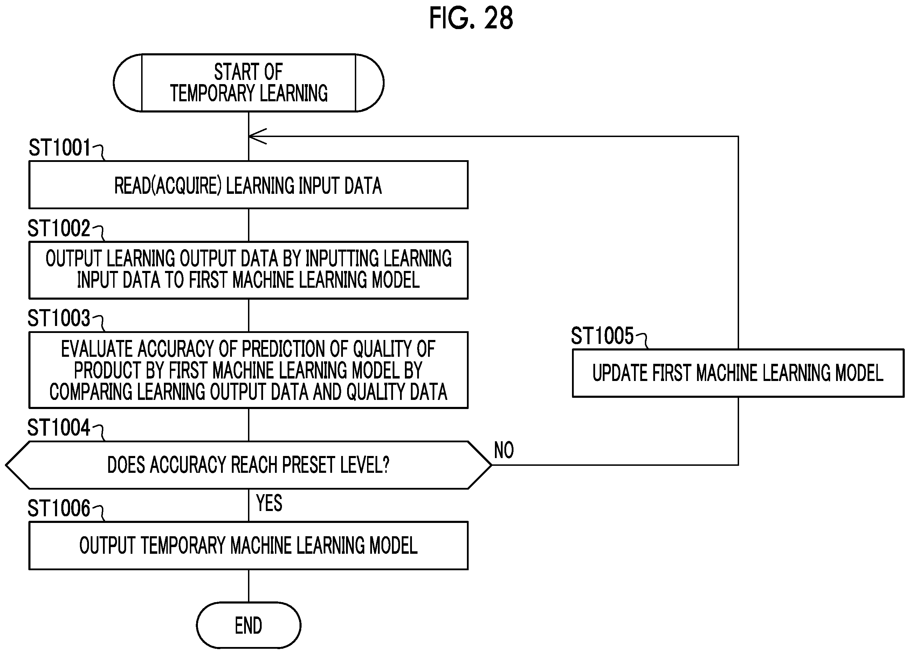

As illustrated in Fig. 28, in the temporary learning, first, the first RW control unit 75 reads the learning input data IDL including a set of the production condition data PCD which is set in the production process and the relevance data PRD, from the storage device 60A (step ST1001). The learning input data IDL is output from the first RW control unit 75 to the learning unit 77. The step ST1001 is an example of a "first acquisition step" according to the technique of the present disclosure.

In the learning unit 77, as illustrated in Fig. 15, the first processing unit 85 outputs the learning output data ODL from the first machine learning model M1 by inputting, as the learning input data IDL, the production condition data PCD and the plurality of items of the relevance data PRD to the first machine learning model M1 without exception (step ST1002). The evaluation unit 86 evaluates the accuracy of prediction of the quality of the product PR by the first machine learning model M1 by comparing the learning output data ODL and the quality data QD (step ST1003).

In a case where an evaluation result of the accuracy of prediction of the first machine learning model M1 by the evaluation unit 86 includes content indicating that the accuracy of prediction of the first machine learning model M1 is lower than a preset level (NO in step ST1004), the update unit 87 updates the first machine learning model M1 (step ST1005). Processing of step ST1001, step ST1002, and step ST1003 is repeated by using the updated first machine learning model M1. In a case where the evaluation result of the accuracy of prediction of the first machine learning model M1 by the evaluation unit 86 includes content indicating that the accuracy of prediction of the first machine learning model M1 reaches a preset level (YES in step ST1004), the processing of step ST1001 to step ST1003 is ended. The first machine learning model M1 of which the accuracy of prediction reaches a preset level is output from the learning unit 77 to the first RW control unit 75, as the temporary machine learning model PM (step ST1006). The temporary machine learning model PM is stored in the storage device 60A by the first RW control unit 75. A series of step ST1001 to step ST1006 is an example of a "temporary learning step" according to the technique of the present disclosure.

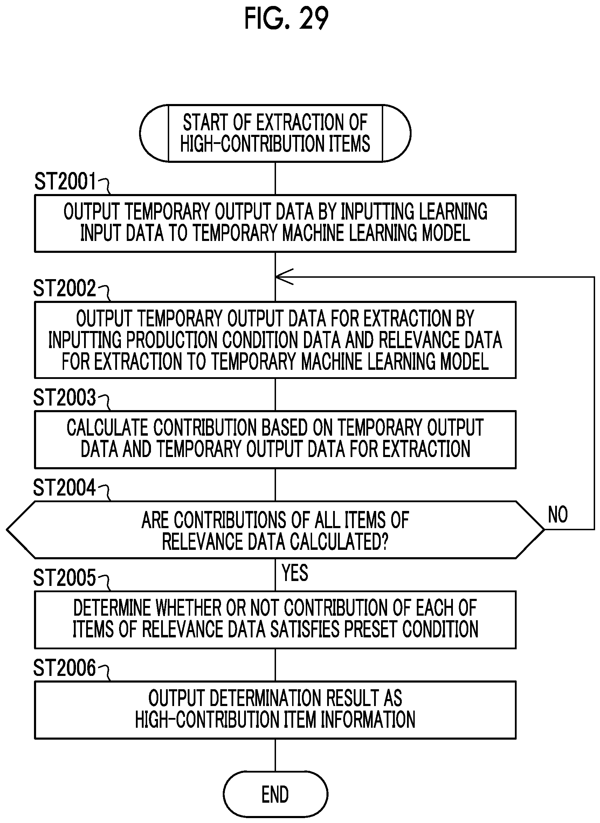

As illustrated in Fig. 29, in extraction of the high-contribution items, as illustrated in Fig. 16 and Fig. 17, the second processing unit 90 outputs the temporary output data POD from the temporary machine learning model PM by inputting the production condition data PCD and the plurality of items of the relevance data PRD to the temporary machine learning model PM without exception (step ST2001). The temporary output data POD is output from the second processing unit 90 to the calculation unit 92.

Further, as illustrated in Fig. 16 and Fig. 18, the third processing unit 91 outputs the temporary output data for extraction PODE from the temporary machine learning model PM by inputting the production condition data PCD and the relevance data for extraction PRDE, which is obtained by excluding one item among the plurality of items of the relevance data PRD, to the temporary machine learning model PM (step ST2002). The temporary output data for extraction PODE is output from the third processing unit 91 to the calculation unit 92.

As illustrated in Fig. 19 and Fig. 20, the calculation unit 92 calculates the contribution of the one item excluded from the relevance data for extraction PRDE based on the temporary output data POD and the temporary output data for extraction PODE (step ST2003). Processing of step ST2002 and step ST2003 is repeatedly performed while changing the items to be excluded one by one until the contributions of all the items of the relevance data PRD are calculated (YES in step ST2004). The contributions of all the items of the relevance data PRD calculated in this way are output from the calculation unit 92 to the determination unit 93, as the contribution information CI illustrated in Fig. 21.

As illustrated in Fig. 22, the determination unit 93 determines whether or not each of the plurality of items of the relevance data PRD is a high-contribution item, based on the contribution information CI and the setting condition SC (step ST2005). The determination result is output from the determination unit 93 to the learning unit 77, as the high-contribution item information HCII (step ST2006). A series of step ST2001 to step ST2006 is an example of an "extraction step" according to the technique of the present disclosure.

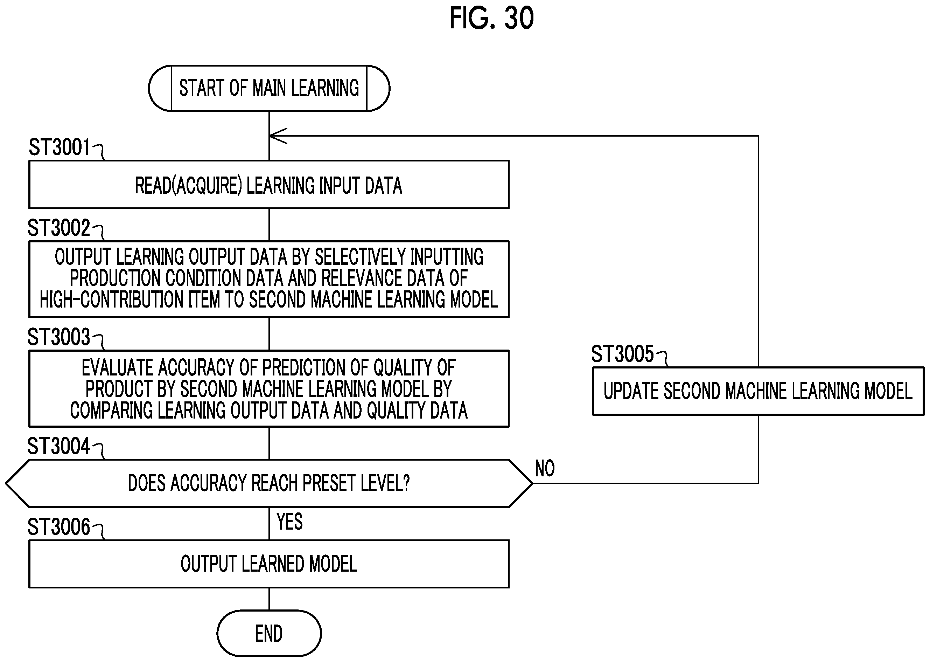

As illustrated in Fig. 30, in the main learning, the first RW control unit 75 reads the learning input data IDL including a set of the production condition data PCD and the relevance data PRD, from the storage device 60A (step ST3001). The learning input data IDL is output from the first RW control unit 75 to the learning unit 77.

In the learning unit 77, as illustrated in Fig. 23, the first processing unit 85 outputs the learning output data ODL from the second machine learning model M2 by selectively inputting, as the learning input data IDL, the production condition data PCD and the relevance data PRD of the high-contribution item to the second machine learning model M2 (step ST3002). The evaluation unit 86 evaluates the accuracy of prediction of the quality of the product PR by the second machine learning model M2 by comparing the learning output data ODL and the quality data QD (step ST3003).

In a case where an evaluation result of the accuracy of prediction of the second machine learning model M2 by the evaluation unit 86 includes content indicating that the accuracy of prediction of the second machine learning model M2 is lower than a preset level (NO in step ST3004), the update unit 87 updates the second machine learning model M2 (step ST3005). Processing of step ST3001, step ST3002, and step ST3003 is repeated by using the updated second machine learning model M2. In a case where the evaluation result of the accuracy of prediction of the second machine learning model M2 by the evaluation unit 86 includes content indicating that the accuracy of prediction of the second machine learning model M2 reaches a preset level (YES in step ST3004), the processing of step ST3001 to step ST3003 is ended. The second machine learning model M2 of which the accuracy of prediction reaches a preset level is output from the learning unit 77 to the first RW control unit 75, as the learned model TM (step ST3006). The learned model TM is stored in the storage device 60Aby the first RW control unit 75. The learned model TM is transmitted to the operating apparatus 11 by the transmission control unit 79. A series of step ST3001 to step ST3006 is an example of a "main learning step" according to the technique of the present disclosure.

In a case where the second operation program 110 is started in the operating apparatus 11, as illustrated in Fig. 24, the CPU 62B of the operating apparatus 11 functions as the second RW control unit 115, the second derivation unit 116, the fourth processing unit 117, and the display control unit 118.

In the operating apparatus 11, the second RW control unit 115 reads the physical-property data for prediction PDF and the high-contribution item information HCII from the storage device 60B, and outputs the physical-property data for prediction PDF and the high-contribution item information HCII to the second derivation unit 116. The second derivation unit 116 derives representative values of the intensity only for the high-contribution item, for each of the plurality of intervals INT1 to INT20 obtained by dividing the spectrum data SPD of the physical-property data for prediction PDF. In this way, the second derivation unit 116 derives the relevance data for prediction PRDF. The relevance data for prediction PRDF is stored in the storage device 60B by the second RW control unit 115.

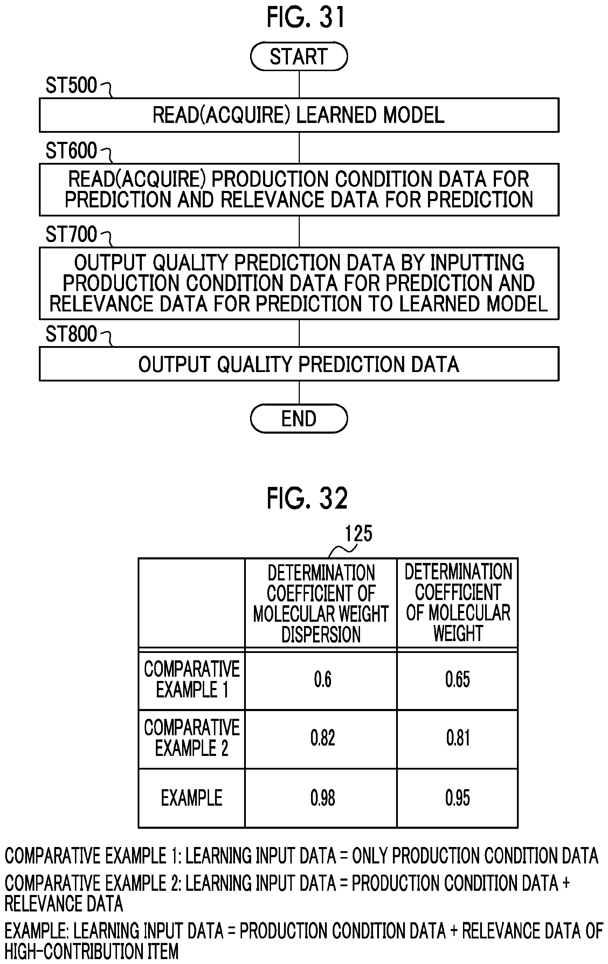

As illustrated in Fig. 31, in the operating apparatus 11, the second RW control unit 115 reads the learned model TM from the storage device 60B (step ST500). Further, the second RW control unit 115 reads, from the storage device 60B, the production condition data for prediction PCDF which is set in the production process and the relevance data for prediction PRDF (step ST600). The learned model TM, the production condition data for prediction PCDF, and the relevance data for prediction PRDF are output from the second RW control unit 115 to the fourth processing unit 117.

As illustrated in Fig. 25, the fourth processing unit 117 outputs the quality prediction data QFD from the learned model TM by inputting the production condition data for prediction PCDF and the relevance data for prediction PRDF to the learned model TM (step ST700). The quality prediction data QFD is output from the fourth processing unit 117 to the display control unit 118.

The display control unit 118 displays the quality prediction display screen 120 illustrated in Fig. 26 on the display 64B (step ST800). The quality prediction display screen 120 allows the operator to browse the quality prediction data QFD.

As described above, in the learning apparatus 10, the first RW control unit 75 acquires the learning input data IDL including the relevance data PRD, and the learning unit 77 performs learning by inputting the learning input data IDL to the first machine learning model M1 and outputs the temporary machine learning model PM. Next, the extraction unit 78 extracts the high-contribution item from the plurality of items of the relevance data PRD by using the temporary machine learning model PM. The learning unit 77 performs learning by selectively inputting the relevance data PRD of the high-contribution item to the second machine learning model M2, and outputs the learned second machine learning model M2 as the learned model TM. Therefore, as compared with a case where the relevance data PRD other than the high-contribution item is input and learned, it is possible to improve the accuracy of prediction of the quality of the product PR by the learned model TM.

More specifically, as illustrated in a table 125 of Fig. 32, in the example in which the production condition data PCD and the relevance data PRD of the high-contribution item are input as the learning input data IDL, a result is obtained that a determination coefficient of the molecular weight dispersion and a determination coefficient of the molecular weight are higher than those of Comparative Example 1 and Comparative Example 2. Comparative Example 1 corresponds to a case where learning is performed by inputting only the production condition data PCD as the learning input data IDL, and Comparative Example 2 corresponds to a case where learning is performed by inputting, as the learning input data IDL, the production condition data PCD and the plurality of items of the relevance data PRD without exception. In this way, in a case where learning is performed by inputting, as the learning input data IDL, the relevance data PRD of the high-contribution item, it is confirmed that the accuracy of prediction of the quality of the product PR by the learned model TM can be further improved.

Further, it is possible to prevent an effort for learning from being spent for the item which has a low contribution in the improvement of the accuracy of prediction, and thus it is possible to improve learning efficiency.

The learning input data IDL includes not only the relevance data PRD but also the production condition data PCD. Thus, the quality prediction data QFD considering an influence of the production condition data PCD can be output from the machine learning model M. Further, the high-contribution item can be extracted in consideration of the influence of the production condition data PCD, and thus validity of the high-contribution item can be further enhanced.

The physical-property data PD includes the spectrum data SPD detected by performing spectroscopic analysis on the product PR. The relevance data PRD is the representative value of the intensity derived for each of the plurality of intervals INT1 to INT20 obtained by dividing the spectrum data SPD. As compared with a case where the intensity of each wave number of the spectrum data SPD is used as the relevance data PRD, a data amount of the relevance data PRD can be reduced.

In the operating apparatus 11, the second RW control unit 115 acquires the learned model TM and the relevance data for prediction PRDF. Next, the fourth processing unit 117 predicts a quality by inputting the relevance data for prediction PRDF to the learned model TM. Under a control of the display control unit 118, the quality prediction display screen 120 including the quality prediction data QFD, which is a quality prediction result by the learned model TM, is displayed on the display 64B. Therefore, the operator can easily recognize a degree of the quality of the product PR without actually applying the product PR to the quality evaluation apparatus 15 and evaluating the quality of the product PR.

Here, in a case where the quality evaluation apparatus 15 actually evaluates the quality of the product PR, it takes a relatively long time, for example, approximately 1 to 2 weeks, for preprocessing and quality evaluation processing of the product PR. On the other hand, in a case where the learned model TM is used, it is possible to predict the quality of the product PR in a very short time. Moreover, as illustrated in Fig. 32, in the example, the accuracy of prediction of the learned model TM is relatively high, and thus the operator can make a future production plan for the product PR with great reference to the quality prediction data QFD.

[Second Embodiment]

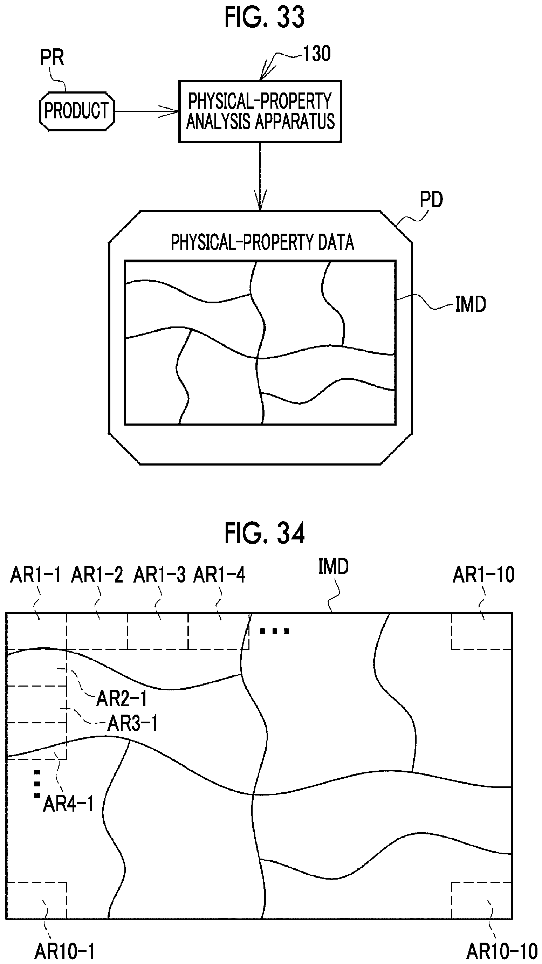

In the second embodiment illustrated in Fig. 33 to Fig. 35, it is assumed that image data IMD obtained by imaging the product PR is the physical-property data PD.

In Fig. 33, the physical-property analysis apparatus 130 according to the second embodiment is, for example, a digital optical microscope, and outputs, as the physical-property data PD, the image data IMD obtained by imaging the product PR. The image data IMD is an example of "multi-dimensional physical-property data" according to the technique of the present disclosure.

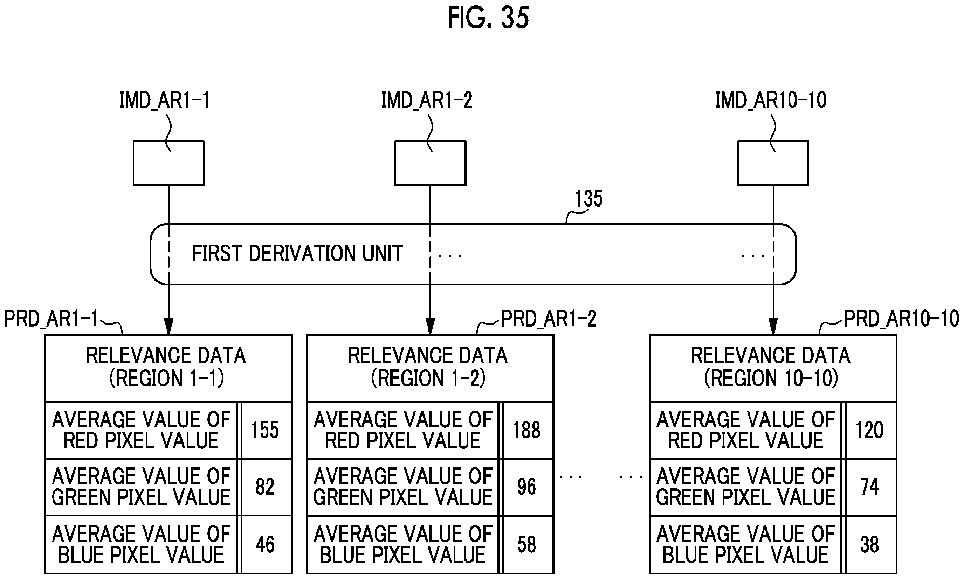

As illustrated in Fig. 34 and Fig. 35, a first derivation unit 135 according to the second embodiment derives pieces of relevance data PRD AR1-1, PRD_AR1-2, ···, and PRD_AR10-10 for each of a plurality of regions AR1-1, AR1-2, ···, and AR10-10 obtained by equally dividing the image data IMD Specifically, the first derivation unit 135 derives, as each of pieces of relevance data PRD_AR1-1 to PRD_AR10-10, an average value of a red pixel value, an average value of a green pixel value, and an average value of a blue pixel value of each of pieces of image data IMD_AR1-1 to IMD_AR10-10 of regions AR1-1 to AR10-10. Instead of or in addition to the average value of each color pixel value, other representative values such as a maximum value, a minimum value, a median value, and a variance of each color pixel value may be derived as the relevance data PRD Further, although an illustration and a detailed description are omitted, similar to the first derivation unit 135, the second derivation unit of the operating apparatus also derives, as relevance data for prediction PRDF, an average value of a red pixel value, an average value of a green pixel value, and an average value of a blue pixel value of each of the regions AR1-1 to AR10-10 of the image data IMD of the product PR of which the quality is unknown.

As described above, in the second embodiment, it is assumed that the image data IMD obtained by imaging the product PR is the physical-property data PD. Therefore, the physical property of the product PR can be more easily recognized.

The image data IMD may be used as the physical-property data PD instead of the spectrum data SPD according to the first embodiment, or may be used as the physical-property data PD in addition to the spectrum data SPD. Further, the physical-property analysis apparatus 130 is not limited to the exemplified digital optical microscope, and may be a scanning electron microscope (SEM) or the like.

The multi-dimensional physical-property data is not limited to the spectrum data SPD according to the first embodiment and the image data IMD according to the second embodiment. The multi-dimensional physical-property data related to any of five human senses of sight, hearing, smell, touch, and taste may be used. For example, touch data of the product PR, odor data at a time of production of the product PR, audio data at a time of production of the product PR, and the like may be used. In a case of touch data, an output of a touch sensor provided at each of a plurality of portions of the product PR is used as touch data, and an average value of the output of the touch sensor provided at each of the plurality of portions is derived as the relevance data PRD. In a case of audio data, an audio recorded for a period from the start to the end of production by a microphone is used as audio data. In this case, the audio data is divided into a plurality of intervals, and an average value of a frequency of each of the plurality of intervals and an average value of an amplitude of each of the plurality of intervals are derived as the relevance data PRD.

[Third Embodiment]

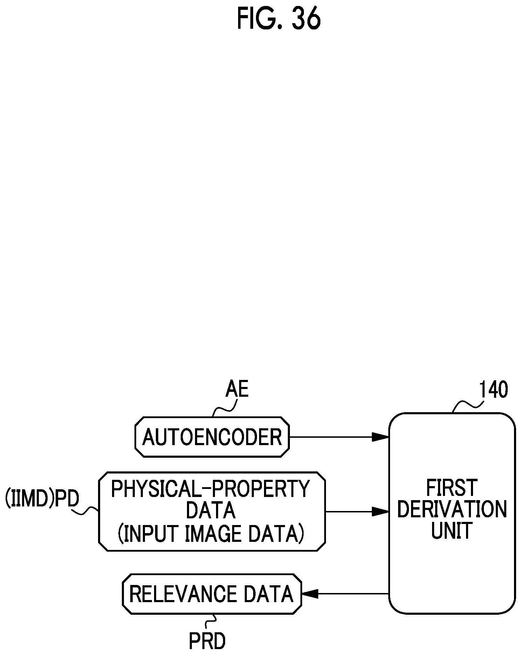

In the third embodiment illustrated in Fig. 36 to Fig. 43, output image data OIMD is output by inputting, as the physical-property data PD, input image data IIMD to an autoencoder AE, and the relevance data PRD is derived based on difference data DD between the input image data IIMD which is input to the autoencoder AE and the output image data OIMD.

In Fig. 36, the first derivation unit 140 according to the third embodiment derives the relevance data PRD from the input image data IIMD as the physical-property data PD by using the autoencoder AE. That is, the first derivation unit 140 is an example of a "derivation unit" according to the technique of the present disclosure. In the following, a case where image data SPIMD of the spectrum SP, which is an example of the "multi-dimensional physical-property data" according to the technique of the present disclosure, is used as the input image data IIMD will be described.

The autoencoder AE is a hierarchical machine learning model configured with a convolutional neural network that includes a plurality of layers for analyzing the input image data IIMD and extracts, for each layer, features in different frequency bands of spatial frequencies included in the input image data IIMD The convolutional neural network is, for example, an U-shaped neural network (U-Net), SegNet, or the like.

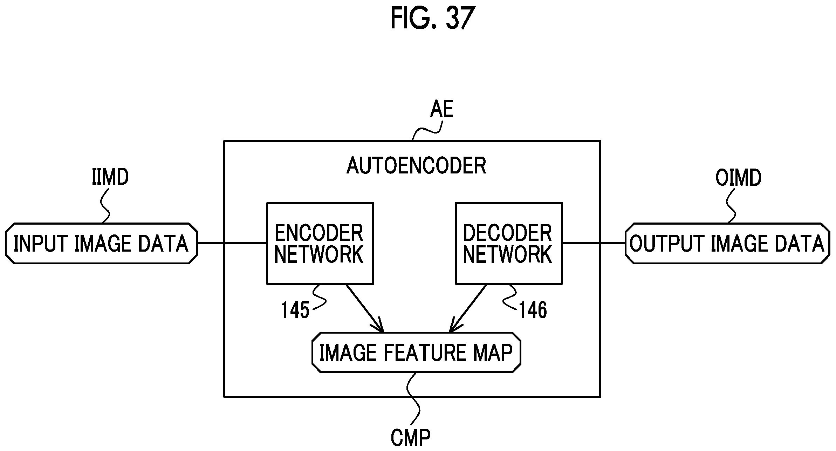

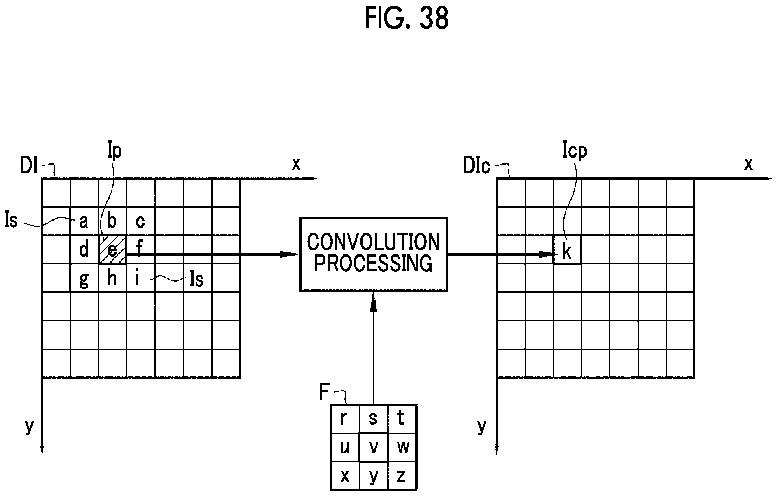

As illustrated in Fig. 37, the autoencoder AE includes an encoder network 145 and a decoder network 146. The encoder network 145 performs convolution processing for extracting a image feature map CMP by performing a convolution operation using a filter F (refer to Fig. 38) for each layer. The decoder network 146 gradually enlarges an image size of a minimum-size image feature map CMP which is output from a lowest layer of the encoder network 145. The image feature map CMP which is gradually enlarged and the image feature map CMP which is output in each layer of the encoder network 145 are combined with each other, and thus output image data OIMD having an image size similar to the image size of the input image data IIMD is generated.

In each layer of the encoder network 145, input data DI (refer to Fig. 38) in which a plurality of pixel values are two-dimensionally arranged is input. In each layer of the encoder network 145, convolution processing is performed on the input data DI, and thus an image feature map CMP is extracted. The input image data IIMD is input to the uppermost first layer of the encoder network 145, as input data DI. In the first layer, convolution processing is performed on the input image data IIMD, and thus, for example, an image feature map CMP having the same image size as the input image data IIMD is output. In the second layer and the lower layers, the image feature map CMP output by each higher layer is input as input data DI. In the second layer and the lower layers, convolution processing is performed on the image feature map CMP, and thus, for example, an image feature map CMP having the same image size as the input image feature map CMP is output.

In Fig. 38, the convolution processing is processing of obtaining output data DIc in which the pixel values are two-dimensionally arranged in the same manner as the input data DI by applying, for example, a $3 \times 3$ filter F to the input data DI, and convolving a pixel value e of an attention pixel Ip in the input data DI and pixel values a, b, c, d, f, g, h, and i of eight pixels Is adjacent to the attention pixel Ip. In a case where coefficients of the filter F are r, s, t, u, v, w, x, y, and z, a pixel value k of a pixel Icp of the output data DIc, which is a result of a convolution operation on the attention pixel Ip, is obtained by, for example, calculating the following Equation 2.

$$k = az + by + cx + dw + ev + fu + gt + hs + ir \cdots \text{(Equation 2)}$$

In the convolution processing, the pixel value k is output by performing the convolution operation on each pixel of the input data DI. In this way, the output data DIc in which the pixel values k are two-dimensionally arranged is output. One piece of the output data DIc is output in correspondence with one filter F. In a case where a plurality of filters F having different types are used, the output data DIc is output for each filter F.

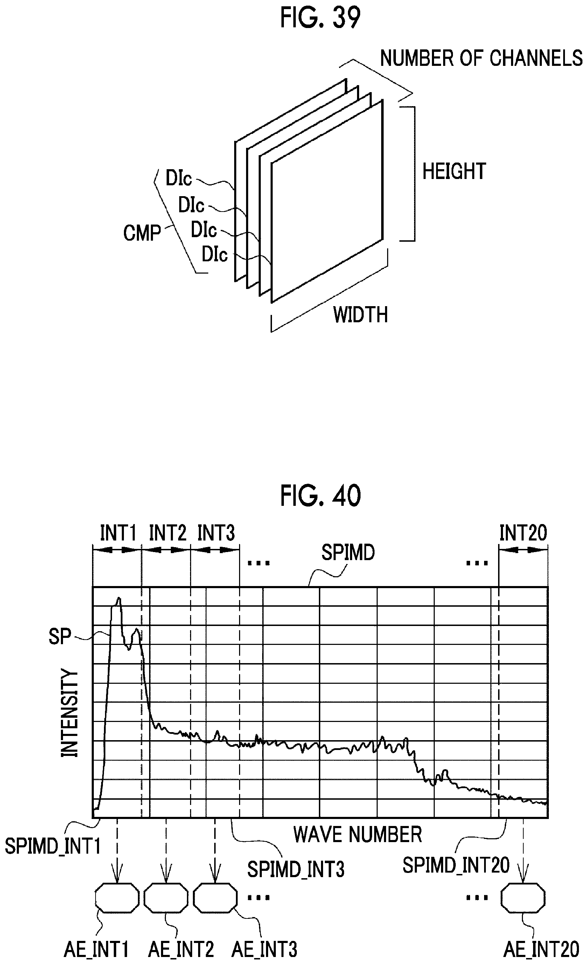

As illustrated in Fig. 39, the output data DIc is data in which the pixel values k are two-dimensionally arranged, and has a width and a height. Further, in a case where a plurality of pieces of output data DIc are output by applying a plurality of filters F having different types, the image feature map CMP is a set of the plurality of pieces of output data DIc. In the image feature map CMP, the number of filters F is called the number of channels. The image feature map CMP illustrated in Fig. 39 is an example of a 4-channel image feature map CMP including four pieces of output data DIc which are output by applying four filters F.

As illustrated in Fig. 40, the autoencoders AE are prepared for each of the plurality of intervals INT1 to INT20 of the spectrum data SPD (autoencoders AE_INT1 to AE_INT20). The autoencoders AE_INT1 to AE_INT20 are specialized for pieces of the image data SPIMD_INT1 to SPIMDINT20 of the spectrum SP in the intervals INT1 to INT20. In the following description, the autoencoders AE_INT1 to AE _INT20 may be collectively referred to as the autoencoder AE.

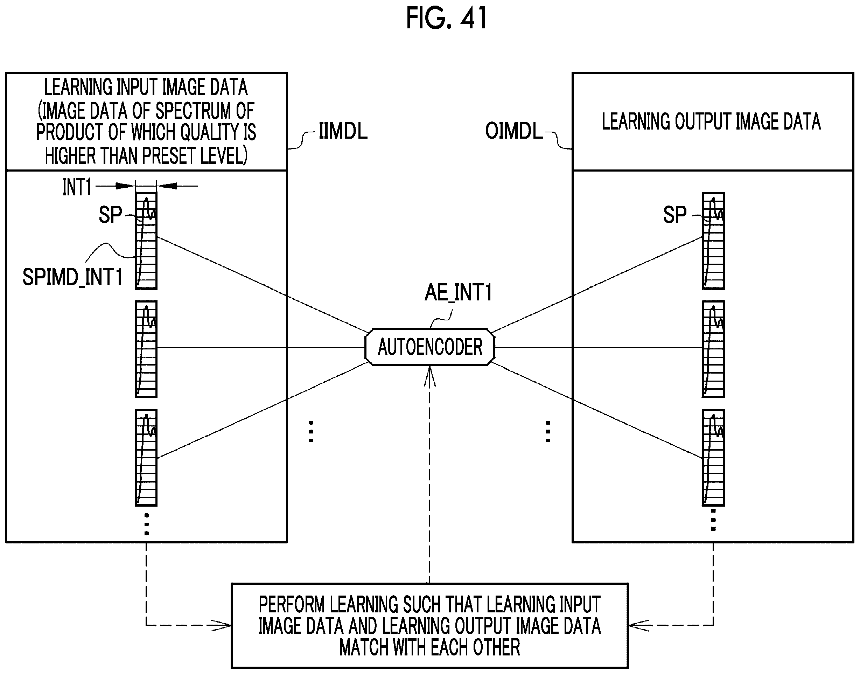

As illustrated in Fig. 41, the image data SPIMD of the spectrum SP of the product PR of which the quality is higher than the preset level is input to the autoencoder AE, as the learning input image data IIMDL. The autoencoder AE is learned such that the learning input image data IIMDL and the learning output image data OIMDL match with each other. That is, in a case where the image data SPIMD of the spectrum SP of the product PR of which the quality is higher than the preset level is input as the input image data IIMD, ideally, the autoencoder AE outputs the output image data OIMD of which the spectrum SP has the same shape as the spectrum SP of the input image data IIMD The preset level means, for example, that the molecular weight dispersion is equal to or higher than 1.5, or that the molecular weight is equal to or larger than 25,000.

Fig. 41 illustrates the autoencoder AE_INT1 of the autoencoders AE_INT1 to AE_INT20. Thus, the learning input image data IIMDL is the image data SPIMD_INT1 of the spectrum SP in the interval INT1.

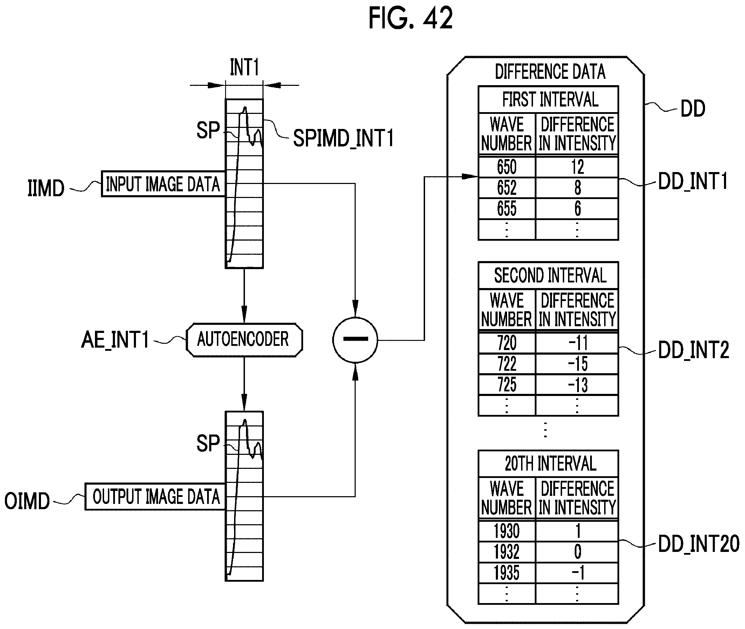

As illustrated in Fig. 42, the first derivation unit 140 outputs the output image data OIMD by inputting, as the input image data IIMD, the image data SPIMD of the spectrum SP to the autoencoder AE. Next, the first derivation unit 140 derives the difference data DD between the input image data IIMD and the output image data OIMD. Specifically, the first derivation unit 140 derives, as the difference data DD, a difference in the intensity of each wave number between the input image data IIMD and the output image data OIMD for each of the intervals INT1 to INT20.

In Fig. 42, as in Fig. 41, only the autoencoder AE_INT1 is illustrated. As the input image data IIMD, the image data SPIMD_INT1 of the spectrum SP in the interval INT1 is input to the autoencoder AE_INT1. The difference data DD_INT1 in the interval INT1 is derived based on the input image data IIMD and the output image data OIMD of the autoencoder AE_INT1.

Here, as illustrated in Fig. 41, the autoencoder AE is learned by inputting, as the learning input image data IIMDL, the image data SPIMD of the spectrum SP of the product PR of which the quality is higher than the preset level. Thus, in a case where the quality of the product PR which is a source of the input image data IIMD is higher than the preset level, the output image data OIMD substantially the same as the input image data IIMD is output from the autoencoder AE. Therefore, the difference in the intensity is relatively small. On the contrary, in a case where the quality of the product PR is equal to or lower than the preset level, the output image data OIMD different from the input image data IIMD is output from the autoencoder AE. Therefore, the difference in the intensity is relatively large. That is, according to the difference data DD, the operator can recognize whether or not the quality of the product PR is higher than the preset level.

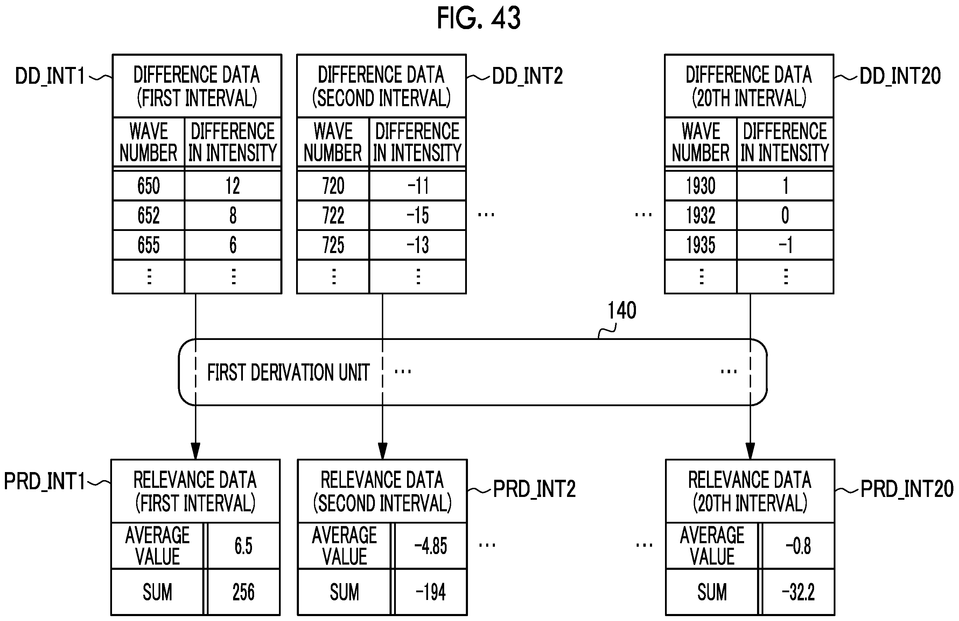

As illustrated in Fig. 43, the first derivation unit 140 derives pieces of the relevance data PRD _INT1, PRD _INT2, ⋯, and PRD _INT20 for each of pieces of the difference data DD_INT1, DD_INT2, ⋯, and DD_INT20 in each of the intervals INT1 to INT20. Specifically, the first derivation unit 140 derives, as pieces of the relevance data PRD_INT1 to PRD _INT20, an average value and a sum of the differences in the intensity in each of the intervals INT1 to INT20. Instead of or in addition to the average value and the sum of the differences in the intensity, other representative values such as a maximum value, a minimum value, a median value, and a variance of the differences in the intensity may be derived as the relevance data PRD. Further, although an illustration and a detailed description are omitted, similar to the first derivation unit 140, the second derivation unit of the operating apparatus also derives, as relevance data for prediction PRDF, the average value and the sum of the differences in the intensity in each of the intervals INT1 to INT20 of the image data SPIMD of the spectrum SP of the product PR of which the quality is unknown.

As described above, in the third embodiment, the first derivation unit 140 outputs the output image data OIMD by inputting, as the physical-property data PD, input image data IIMD to the autoencoder AE, and derives the relevance data PRD based on the difference data DD between the input image data IIMD which is input to the autoencoder AE and the output image data OIMD. Thus, as compared with the representative values of the intensity, such as the average value, the maximum value, the minimum value, the median value, the variance, the skewness, and the kurtosis which are exemplified in the first embodiment, numerical values that more accurately represent the physical property of the product PR (in the embodiment, the average value and the sum of the differences in the intensity) can be derived as the relevance data PRD. Therefore, the accuracy of prediction of the learned model TM can be further improved.

The relevance data PRD based on the difference data DD may be used instead of or in addition to the representative values of the intensity, such as the average value, the maximum value, the minimum value, the median value, the variance, the skewness, and the kurtosis which are exemplified in the first embodiment.

The input image data IIMD is not limited to the image data SPIMD of the exemplified spectrum SP. Instead of or in addition to the image data SPIMD of the spectrum SP, the image data IMD according to the second embodiment that is obtained by imaging the product PR may be used as the input image data IIMD In this case, the autoencoder AE is prepared for each region AR of the image data IMD.

[Fourth Embodiment]

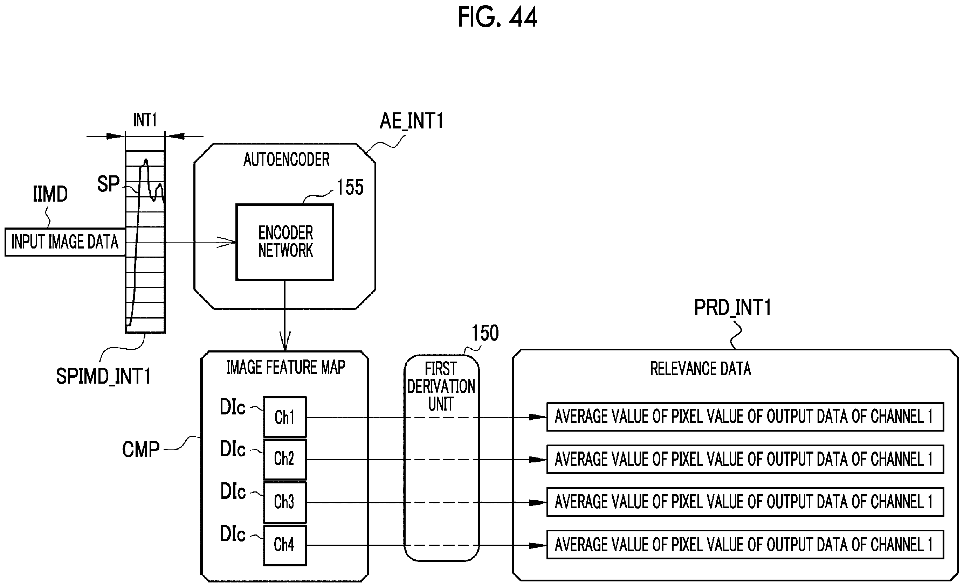

In the fourth embodiment illustrated in Fig. 44, the relevance data PRD is derived based on the image feature map CMP output from an encoder network 155 which is a part of the autoencoder AE. That is, the image feature map CMP is an example of "feature data" according to the technique of the present disclosure.

As illustrated in Fig. 44, similar to the third embodiment, the first derivation unit 150 according to the fourth embodiment inputs, as the input image data IIMD, the image data SPIMD of the spectrum SP which is the physical-property data PD, to the autoencoder AE. The image feature map CMP is output from the encoder network 155 of the autoencoder AE. The image feature map CMP is, for example, a minimum-size image feature map CMP which is output from a lowest layer of the encoder network 155. The autoencoder AE according to the fourth embodiment is prepared for each of the plurality of intervals INT1 to INT20 of the spectrum data SPD, similar to the autoencoder AE according to the third embodiment. Here, unlike the autoencoder AE according to the third embodiment, the autoencoder AE according to the fourth embodiment is learned by inputting, as the learning input image data IIMDL, the image data SPIMD of the spectrum SP of various products PR regardless of the level of the quality.

The first derivation unit 150 derives the relevance data PRD based on the image feature map CMP. Fig. 44 illustrates a case where the image feature map CMP includes the output data DIc of a channel 1 (Ch1), the output data DIc of a channel 2 (Ch2), the output data DIc of a channel 3 (Ch3), and the output data DIc of a channel 4 (Ch4). An example in which the first derivation unit 150 derives, as the relevance data PRD, an average value of a pixel value for each of pieces of the output data DIc of the channels 1 to 4 is illustrated. Instead of or in addition to the average value, a maximum value, a minimum value, a median value, a variance, or the like may be derived as the relevance data PRD. Further, although an illustration and a detailed description are omitted, similar to the first derivation unit 150, the second derivation unit of the operating apparatus also derives, as relevance data for prediction PRDF, an average value of a pixel value of the output data DIc of the image feature map CMP in the intervals INT1 to INT20 of the image data SPIMD of the spectrum SP of the product PR of which the quality is unknown.

In Fig. 44, only the autoencoder AE_INT1 is illustrated. As the input image data IIMD, the image data SPIMD_INT1 of the spectrum SP in the interval INT1 is input to the autoencoder AE_INT1. The first derivation unit 150 derives the relevance data PRD_INT1 in the interval INT1, from the image feature map CMP of the encoder network 155 of the autoencoder AE_INT1. Although an illustration and a description are omitted, pieces of the relevance data PRD_INT2 to PRD _INT20 in other intervals INT2 to INT20 are also derived from the image feature map CMP of the encoder network 155 of each of the autoencoders AE_INT2 to AE_INT20.

As described above, in the fourth embodiment, the relevance data PRD is derived based on the image feature map CMP output from the encoder network 155 of the autoencoder AE. Therefore, as in the third embodiment, a numerical value (in the example, the average value of the pixel value of the output data DIc) that accurately represents the physical property of the product PR can be derived as the relevance data PRD, and thus it is possible to further improve the accuracy of prediction of the learned model TM.

The image feature map CMP may be output from a layer other than the lowest layer of the encoder network 155.

The relevance data PRD based on the image feature map CMP may be used instead of or in addition to the representative values of the intensity, such as the average value, the maximum value, the minimum value, the median value, the variance, the skewness, and the kurtosis which are exemplified in the first embodiment. Further, the relevance data PRD based on the image feature map CMP may be used instead of or in addition to the relevance data PRD based on the difference data DD according to the third embodiment.

In the fourth embodiment, as in the third embodiment, the input image data IIMD is not limited to the image data SPIMD of the exemplified spectrum SP. Instead of or in addition to the image data SPIMD of the spectrum SP, the image data IMD according to the second embodiment that is obtained by imaging the product PR may be used as the input image data IIMD. In this case, the autoencoder AE is prepared for each region AR of the image data IMD.

The intervals INT obtained by dividing the spectrum data SPD may be overlapped with each other. Similarly, the regions AR obtained by dividing the image data IMD may also be overlapped with each other.

The product PR is not limited to the product that is produced by using a flow synthesis method. For example, the product may be produced by using a batch combination method.

In each of the embodiments, the production condition which is received by the setting unit 25 of the flow reaction apparatus 13 is used as the production condition data PCD. On the other hand, the present disclosure is not limited thereto. As the production condition data PCD, actual measurement values that are measured by the first flow velocity sensor 35, the second flow velocity sensor 36, the third flow velocity sensor 37, the temperature sensor 38, the first flow velocity sensor 51, the second flow velocity sensor 52, the third flow velocity sensor 53, and the fourth flow velocity sensor 54 may be used.

In each of the embodiments, the quality prediction display screen 120 is exemplified as an output form of the quality prediction data QFD. On the other hand, the present disclosure is not limited thereto. Instead of or in addition to the quality prediction display screen 120, a form in which the quality prediction data QFD is printed and output on a paper medium and a form in which the quality prediction data QFD is output as a data file may be adopted.

As the machine learning model M, there are machine learning models using linear regression, Gaussian process regression, support vector regression, decision tree, an ensemble method, a bagging method, a boosting method, a gradient boosting method, and the like. Further, there are machine learning models using a simple perceptron, a multi-layer perceptron, a deep neural network, a convolutional neural network, a deep belief network, a recurrent neural network, a stochastic neural network, and the like. Which machine learning model M is used among the above-described models is not particularly limited, and a machine learning model M using any method may be selected.

As an ensemble method, there is random forest. As well known, random forest is a method of improving accuracy of prediction by creating a plurality of decision tree groups with low correlation using randomly-sampled learning data and randomly-selected explanatory variables and integrating and averaging prediction results by the decision tree groups. In this case, control parameters of the machine learning model M include the number of explanatory variables to be selected and the number of branches of the decision trees.

Since the deep neural network has the relatively large number of control parameters, flexible combinations may be made. For this reason, the deep neural network can exhibit high prediction performance for various data structures. The control parameters include the number of layers of the network and the number of nodes of the network, a type of an activated function, a dropout ratio, a mini-batch size, the number of epochs, a learning rate, and the like.

The machine learning model M includes a plurality of execution frameworks, and an execution framework may be appropriately selected from the execution frameworks. For example, an execution framework may be selected from Tensorflow, Cognitive Toolkit (CNTK), Theano, Caffe, mxnet, Keras, PyTorch, Chainer, Scikit-learn, Caret, MATLAB (registered trademark), and the like.

The hardware configuration of the computer including the machine learning system 2 may be modified in various ways. For example, the learning apparatus 10 and the operating apparatus 11 may be integrated and configured by one computer. Further, at least one of the learning apparatus 10 or the operating apparatus 11 may be configured by a plurality of computers which are separated as hardware for the purpose of improving processing capability and reliability. For example, in the learning apparatus 10, the function of the first derivation unit 76, the function of the learning unit 77, and the function of the extraction unit 78 are distributed to three computers. In this case, the learning apparatus 10 is configured by three computers.

In this way, the hardware configuration of the computer of the machine learning system 2 may be appropriately changed according to the required performance such as processing capability, safety, and reliability. Further, not only hardware but also the application program such as the first operation program 70 or the second operation program 110 may be duplicated or distributed and stored in a plurality of storage devices for the purpose of ensuring safety and reliability.

In each of the embodiments, for example, as a hardware structure of the processing unit that executes various processing, such as the first RW control unit 75, the first derivation units 76, 135, 140, and 150, the learning unit 77 (the first processing unit 85, the evaluation unit 86, and the update unit 87), the extraction unit 78 (the second processing unit 90, the third processing unit 91, the calculation unit 92, and the determination unit 93), the transmission control unit 79, the second RW control unit 115, the second derivation unit 116, the fourth processing unit 117, and the display control unit 118, the following various processors may be used. The various processors include, as described above, the CPUs 62A and 62B which are general-purpose processors that function as various processing units by executing software (the first operation program 70 and the second operation program 110), a programmable logic device (PLD) such as a field programmable gate array (FPGA) which is a processor capable of changing a circuit configuration after manufacture, a dedicated electric circuit such as an application specific integrated circuit (ASIC) which is a processor having a circuit configuration specifically designed to execute specific processing, and the like.

One processing unit may be configured by one of these various processors, or may be configured by a combination of two or more processors having the same type or different types (for example, a combination of a plurality of FPGAs and/or a combination of a CPU and an FPGA). Further, the plurality of processing units may be configured by one processor.

As an example in which the plurality of processing units are configured by one processor, firstly, as represented by a computer such as a client and a server, a form in which one processor is configured by a combination of one or more CPUs and software and the processor functions as the plurality of processing units may be adopted. Secondly, as typified by system on chip (System On Chip: SoC), there is a form in which a processor that realizes the functions of the entire system including a plurality of processing units with one IC (Integrated Circuit) chip is used. As described above, the various processing units are configured by using one or more various processors as a hardware structure.

Further, as the hardware structure of the various processors, more specifically, an electric circuit (circuitry) in which circuit elements such as semiconductor elements are combined may be used.

From the above description, the invention described in following Appendixes 1 and 2 can be understood.

[Appendix 1]

A learning apparatus including:

a first acquisition processor configured to acquire learning input data to be input to a machine learning model for predicting a quality of a product, the learning input data including multi-dimensional physical-property relevance data which is derived from multi-dimensional physical-property data representing a physical property of the product and includes a plurality of items;

a temporary learning processor configured to perform learning by inputting the learning input data to the machine learning model and output a temporary machine learning model;

an extraction processor configured to extract, from the plurality of items of the multi-dimensional physical-property relevance data, by using the temporary machine learning model, a high-contribution item of which a contribution to improvement of accuracy of prediction of the quality satisfies a preset condition; and

a main learning processor configured to perform learning by selectively inputting the multi-dimensional physical-property relevance data of the high-contribution item to the machine learning model and output the machine learning model as a learned model to be provided for actual operation.

[Appendix 2]

An operating apparatus including:

a second acquisition processor configured to acquire the learned model which is output from the main learning processor of the learning apparatus according to Appendix 1;

a third acquisition processor configured to acquire multi-dimensional physical-property relevance data for prediction which is data of a product of which a quality is unknown;

a processing processor configured to predict the quality by inputting, to the learned model acquired by the second acquisition processor, the multi-dimensional physical-property relevance data for prediction which is acquired by the third acquisition processor; and

an output control processor configured to control outputting of a prediction result of the quality by the learned model.

The technique of the present disclosure can also appropriately combine the various embodiments and the various modification examples. In addition, the technique of the present disclosure is not limited to each embodiment, and various configurations may be adopted without departing from the scope of the present disclosure. Further, the technique of the present disclosure extends to a program and a storage medium for non-temporarily storing the program.

The described contents and the illustrated contents are detailed explanations of a part according to the technique of the present disclosure, and are merely examples of the technique of the present disclosure. For example, the descriptions related to the configuration, the function, the operation, and the effect are descriptions related to examples of a configuration, a function, an operation, and an effect of a part according to the technique of the present disclosure. Therefore, it goes without saying that, in the described contents and illustrated contents, unnecessary parts may be deleted, new components may be added, or replacements may be made without departing from the spirit of the technique of the present disclosure. Further, in order to avoid complications and facilitate understanding of the part according to the technique of the present disclosure, in the described contents and illustrated contents, descriptions of technical knowledge and the like that do not require particular explanations to enable implementation of the technique of the present disclosure are omitted.

In this specification, "A and/or B" is synonymous with "at least one of A or B". That is, "A and/or B" means that only A may be included, that only B may be included, or that a combination of A and B may be included. Further, in this specification, even in a case where three or more matters are expressed by being connected using "and/or", the same concept as "A and/or B" is applied.

All documents, patent applications, and technical standards mentioned in this specification are incorporated herein by reference to the same extent as in a case where each document, each patent application, and each technical standard are specifically and individually described by being incorporated by reference.

Explanation of References

2: machine learning system
10: learning apparatus
11: operating apparatus
12: network
13: flow reaction apparatus
14, 130: physical-property analysis apparatus
15: quality evaluation apparatus
20: first raw material supply unit
21: second raw material supply unit
22, 45: reaction section

23: temperature control unit

24: recovery/discard section

25: setting unit

26: system controller

30, 46: junction portion

31: reaction portion

32 to 34: first to third pipe portions

35 to 37: first flow velocity sensor to third flow velocity sensor

38: temperature sensor

40: recovery unit

41: discard unit

42: three-way valve

47 to 50: first to fourth pipe portions

51 to 54: first flow velocity sensor to fourth flow velocity sensor

60, 60A, 60B: storage device

61: memory

62, 62A, 62B: CPU

63: communication unit

64, 64B: display

65, 65B: input device

66: bus line

70: first operation program (operation program of learning apparatus)

75: first read/write control unit (first RW control unit, first acquisition unit)

76, 135, 140, 150: first derivation unit (derivation unit)

77: learning unit (temporary learning unit, main learning unit)

78: extraction unit

79: transmission control unit

85: first processing unit

86: evaluation unit

87: update unit

90: second processing unit

91: third processing unit

92: calculation unit

93: determination unit

100: conversion table

110: second operation program

115: second read/write control unit (second RW control unit, second acquisition unit, third acquisition unit)

116: second derivation unit

117: fourth processing unit (processing unit)

118: display control unit (output control unit)

120: quality prediction display screen

121: OK button

125: table

145, 155: encoder network

146: decoder network

AE: autoencoder

AR: region

CI: contribution information

CMP: image feature map

DD: difference data

DI: input data of autoencoder

DIc: output data of autoencoder

F: filter

HCII: high-contribution item information

Icp: pixel of output data

IDL: learning input data

IIMD: input image data

IIMDL: learning input image data

IMD: image data
INT: interval
Ip: attention pixel
Is: adjacent pixel
L: length of reaction path
M: machine learning model
M1, M2: first machine learning model, second machine learning model
ODL: learning output data
OIMD: output image data
OIMDL: learning output image data
PCD: production condition data
PCDF: production condition data for prediction
PD: physical-property data
PDF: physical-property data for prediction
PM: temporary machine learning model
POD: temporary output data
PODE: temporary output data for extraction
PR: product
PRD: physical-property relevance data (relevance data)
PRDE: physical-property relevance data for extraction (relevance data for extraction)
PRDF: physical-property relevance data for prediction (relevance data for prediction)
QD: quality data
QFD: quality prediction data
RM: raw material
RM1, RM2: first raw material, second raw material
SC: setting condition
SP: spectrum
SPD: spectrum data
SPIMD: image data of spectrum
ST100, ST200, ST300, ST500, ST600, ST700, ST800: step
ST1001 to ST1006: step (temporary learning step)
ST2001 to ST2006: step (extraction step)
ST3001 to ST3006: step (main learning step)
TM: learned model
Ø: diameter of reaction path

**Claims**

1. A learning apparatus comprising:

   a first acquisition unit that acquires learning input data to be input to a machine learning model for predicting a quality of a product, the learning input data including multi-dimensional physical-property relevance data which is derived from multi-dimensional physical-property data representing a physical property of the product and includes a plurality of items;
   a temporary learning unit that inputs the learning input data to the machine learning model, performs learning, and outputs a temporary machine learning model;
   an extraction unit that extracts a high-contribution item from the plurality of items of the multi-dimensional physical-property relevance data by using the temporary machine learning model, the high-contribution item being the item of which a contribution to improvement of accuracy of prediction of the quality satisfies a preset condition; and
   a main learning unit that selectively inputs the multi-dimensional physical-property relevance data of the high-contribution item to the machine learning model, performs learning, and outputs the machine learning model as a learned model to be provided for actual operation.

2. The learning apparatus according to claim 1,
   wherein the learning input data includes production condition data which is set in a production process of the product.

3. The learning apparatus according to claim 1 or 2,
   wherein the multi-dimensional physical-property data includes spectrum data which is detected by performing spectroscopic analysis on the product.

4. The learning apparatus according to claim 3,
   wherein the multi-dimensional physical-property relevance data is a representative value of an intensity derived for each of a plurality of intervals obtained by dividing the spectrum data.

5. The learning apparatus according to any one of claims 1 to 4,
   wherein the multi-dimensional physical-property data includes image data obtained by imaging the product.

6. The learning apparatus according to any one of claims 1 to 5,
   wherein the product is produced by using a flow synthesis method.

7. The learning apparatus according to any one of claims 1 to 6, further comprising:
   a derivation unit that derives the multi-dimensional physical-property relevance data by applying at least a part of an autoencoder to the multi-dimensional physical-property data.

8. The learning apparatus according to claim 7,

   wherein the autoencoder is learned by inputting the multi-dimensional physical-property data of the product of which the quality is higher than a preset level, and
   wherein the derivation unit inputs the multi-dimensional physical-property data to the autoencoder, outputs output data, and derives the multi-dimensional physical-property relevance data based on difference data between the multi-dimensional physical-property data which is input to the autoencoder and the output data.

9. The learning apparatus according to claim 7 or 8,
   wherein the derivation unit inputs the multi-dimensional physical-property data to the autoencoder, outputs feature data from an encoder network of the autoencoder, and derives the multi-dimensional physical-property relevance data based on the feature data.

10. The learning apparatus according to any one of claims 7 to 9,
    wherein the multi-dimensional physical-property data includes image data of a spectrum which is represented by spectrum data detected by performing spectroscopic analysis on the product.

11. The learning apparatus according to claim 10,
    wherein the derivation unit derives the multi-dimensional physical-property relevance data for each of a plurality of intervals obtained by dividing the spectrum data.

12. An operating apparatus comprising:

    a second acquisition unit that acquires the learned model which is output from the main learning unit of the learning apparatus according to any one of claims 1 to 11;
    a third acquisition unit that acquires multi-dimensional physical-property relevance data for prediction which is data of a product of which a quality is unknown;
    a processing unit that inputs the multi-dimensional physical-property relevance data for prediction which is data of the product of which the quality is unknown, which is acquired by the third acquisition unit, to the learned model acquired by the second acquisition unit, and predicts the quality; and
    an output control unit that controls outputting of a prediction result of the quality by the learned model.

13. An operation method of a learning apparatus, the operation method comprising:

    a first acquisition step of acquiring learning input data to be input to a machine learning model for predicting a quality of a product, the learning input data including multi-dimensional physical-property relevance data which is derived from multi-dimensional physical-property data representing a physical property of the product and includes a plurality of items;
    a temporary learning step of inputting the learning input data to the machine learning model, performing learning, and outputting a temporary machine learning model;

an extraction step of extracting a high-contribution item from the plurality of items of the multi-dimensional physical-property relevance data by using the temporary machine learning model, the high-contribution item being the item of which a contribution to improvement of accuracy of prediction of the quality satisfies a preset condition; and

a main learning step of selectively inputting the multi-dimensional physical-property relevance data of the high-contribution item to the machine learning model, performing learning, and outputting the machine learning model as a learned model to be provided for actual operation.

**14.** An operation program of a learning apparatus, the program causing a computer to function as:

a first acquisition unit that acquires learning input data to be input to a machine learning model for predicting a quality of a product, the learning input data including multi-dimensional physical-property relevance data which is derived from multi-dimensional physical-property data representing a physical property of the product and includes a plurality of items;

a temporary learning unit that inputs the learning input data to the machine learning model, performs learning, and outputs a temporary machine learning model;

an extraction unit that extracts a high-contribution item from the plurality of items of the multi-dimensional physical-property relevance data by using the temporary machine learning model, the high-contribution item being the item of which a contribution to improvement of accuracy of prediction of the quality satisfies a preset condition; and

a main learning unit that selectively inputs the multi-dimensional physical-property relevance data of the high-contribution item to the machine learning model, performs learning, and outputs the machine learning model as a learned model to be provided for actual operation.

# FIG. 1

# FIG. 2

# FIG. 3

LEARNING APPARATUS

PRODUCTION CONDITION DATA — PCD

PHYSICAL-PROPERTY DATA — PD

QUALITY DATA — QD

PHYSICAL-PROPERTY RELEVANCE DATA — IDL

PRD    M

MACHINE LEARNING MODEL

LEARNING OUTPUT DATA — ODL

UPDATE

EVALUATION

10

LEARNED MODEL — TM

11

OPERATING APPARATUS

PCDF    PRDF

PRODUCTION CONDITION DATA FOR PREDICTION

PHYSICAL-PROPERTY RELEVANCE DATA FOR PREDICTION

LEARNED MODEL — TM

QUALITY PREDICTION DATA — QFD

EP 3 995 996 A1

FIG. 4

FIG. 5

# FIG. 6

TO LEARNING APPARATUS

TO PHYSICAL-PROPERTY ANALYSIS APPARATUS AND QUALITY EVALUATION APPARATUS

20 — FIRST RAW MATERIAL SUPPLY UNIT

RM — FIRST RAW MATERIAL

RM1

PCD — 26

25 — SETTING UNIT

PRODUCTION CONDITION DATA

SYSTEM CONTROLLER

RM2

SECOND RAW MATERIAL SUPPLY UNIT — 21

SECOND RAW MATERIAL

TEMPERATURE CONTROL UNIT — 23

PR — PRODUCT

42 — 40

RECOVERY UNIT

DISCARD UNIT

RECOVERY/DISCARD SECTION 41

24

FLOW REACTION APPARATUS

51  47  45  46  31  49  54  53  52  48  50  38  L  13  φ

EP 3 995 996 A1

## FIG. 7

PCD

PRODUCTION CONDITION DATA

| CONCENTRATION OF FIRST RAW MATERIAL (mol/l) | FLOW VELOCITY OF FIRST RAW MATERIAL (ml/min) | CONCENTRATION OF SECOND RAW MATERIAL (mol/l) | FLOW VELOCITY OF SECOND RAW MATERIAL (ml/min) | SHAPE OF JUNCTION PORTION | DIAMETER OF REACTION PATH (mm) | LENGTH OF REACTION PATH (m) | REACTION PATH TEMPERATURE (°C) |
|---|---|---|---|---|---|---|---|
| 0.018 | 10 | 0.018 | 5.5 | T-SHAPE | 5 | 8 | 10 |

# FIG. 8

PR

PRODUCT → PHYSICAL-PROPERTY ANALYSIS APPARATUS ~14

PHYSICAL-PROPERTY DATA    PD

| SPECTRUM DATA | |
|---|---|
| WAVE NUMBER | INTENSITY |
| 650 | 45 |
| 652 | 110 |
| 655 | 224 |
| ⋮ | ⋮ |
| 1000 | 385 |
| 1004 | 386 |
| ⋮ | ⋮ |
| 1995 | 88 |
| 2000 | 87 |

SPD

SP

INTENSITY

WAVE NUMBER

# FIG. 9

PR

PRODUCT → QUALITY EVALUATION APPARATUS ~15

QUALITY DATA QD

| MOLECULAR WEIGHT DISPERSION | MOLECULAR WEIGHT |
|---|---|
| 1.0422 | 22000 |

# FIG. 10

10,11

- DISPLAY ~64
- INPUT DEVICE ~65
- CPU ~62
- MEMORY ~61
- STORAGE DEVICE ~60
- COMMUNICATION UNIT ~63

66

12

# FIG. 11

# FIG. 12

## FIG. 13

| SPD_INT1 | SPECTRUM DATA (FIRST INTERVAL) | | SPECTRUM DATA (SECOND INTERVAL) | | SPD_INT2 | SPECTRUM DATA (20TH INTERVAL) | | SPD_INT20 |
|---|---|---|---|---|---|---|---|---|
| | WAVE NUMBER | INTENSITY | WAVE NUMBER | INTENSITY | | WAVE NUMBER | INTENSITY | |
| | 650 | 45 | 720 | 2040 | ··· ··· | 1930 | 45 | |
| | 652 | 110 | 722 | 2035 | | 1932 | 110 | |
| | 655 | 224 | 725 | 2028 | | 1935 | 224 | |
| | ⋮ | ⋮ | ⋮ | ⋮ | | ⋮ | ⋮ | |

76

FIRST DERIVATION UNIT ···  ···

| PRD_INT1 | RELEVANCE DATA (FIRST INTERVAL) | | RELEVANCE DATA (SECOND INTERVAL) | | PRD_INT2 | RELEVANCE DATA (20TH INTERVAL) | | PRD_INT20 |
|---|---|---|---|---|---|---|---|---|
| | AVERAGE VALUE | 1500 | AVERAGE VALUE | 1960 | | AVERAGE VALUE | 360 | |
| | MAXIMUM VALUE | 2800 | MAXIMUM VALUE | 2055 | | MAXIMUM VALUE | 421 | |
| | MINIMUM VALUE | 45 | MINIMUM VALUE | 1876 | ··· ··· | MINIMUM VALUE | 126 | |
| | MEDIAN VALUE | 1150 | MEDIAN VALUE | 1921 | | MEDIAN VALUE | 285 | |
| | VARIANCE | 60.95 | VARIANCE | 5.03 | | VARIANCE | 18.29 | |
| | SKEWNESS | 0.985 | SKEWNESS | 0.997 | | SKEWNESS | 0.123 | |
| | KURTOSIS | 16.84 | KURTOSIS | 4.69 | | KURTOSIS | −12.4 | |

EP 3 995 996 A1

# FIG. 14

EP 3 995 996 A1

# FIG. 15

⟨TEMPORARY LEARNING⟩

**ID: PR0001** — PCD

### PRODUCTION CONDITION DATA

| CONCENTRATION OF FIRST RAW MATERIAL (mol/l) | FLOW VELOCITY OF FIRST RAW MATERIAL (ml/min) | CONCENTRATION OF SECOND RAW MATERIAL (mol/l) | FLOW VELOCITY OF SECOND RAW MATERIAL (ml/min) | SHAPE OF JUNCTION PORTION | DIAMETER OF REACTION PATH (mm) | LENGTH OF REACTION PATH (m) | REACTION TEMPERATURE (°C) |
|---|---|---|---|---|---|---|---|
| 0.018 | 10 | 0.018 | 5.5 | T-SHAPE | 5 | 8 | 10 |

— IDL

**ID: PR0001** — PRD

### RELEVANCE DATA

| FIRST INTERVAL | | | SECOND INTERVAL | | | 20TH INTERVAL | |
|---|---|---|---|---|---|---|---|
| AVERAGE VALUE | 1500 | | AVERAGE VALUE | 1960 | | AVERAGE VALUE | 360 |
| MAXIMUM VALUE | 2800 | | MAXIMUM VALUE | 2055 | | MAXIMUM VALUE | 421 |
| MINIMUM VALUE | 45 | | MINIMUM VALUE | 1876 | | MINIMUM VALUE | 126 |
| MEDIAN VALUE | 1150 | · · · | MEDIAN VALUE | 1921 | · · · | MEDIAN VALUE | 285 |
| VARIANCE | 60.95 | | VARIANCE | 5.03 | | VARIANCE | 18.29 |
| SKEWNESS | 0.985 | | SKEWNESS | 0.997 | | SKEWNESS | 0.123 |
| KURTOSIS | 16.84 | | KURTOSIS | 4.69 | | KURTOSIS | −12.4 |

— PRD_INT1   — PRD_INT2   — PRD_INT20

FIRST MACHINE LEARNING MODEL — M1

85

FIRST PROCESSING UNIT

ODL

### LEARNING OUTPUT DATA

| MOLECULAR WEIGHT DISPERSION | MOLECULAR WEIGHT |
|---|---|
| 1.4588 | 28500 |

FIG. 16
EXTRACTION UNIT

# FIG. 17

ID: PR0100 ~PCD

**PRODUCTION CONDITION DATA**

| CONCENTRATION OF FIRST RAW MATERIAL (mol/l) | FLOW VELOCITY OF FIRST RAW MATERIAL (ml/min) | CONCENTRATION OF SECOND RAW MATERIAL (mol/l) | FLOW VELOCITY OF SECOND RAW MATERIAL (ml/min) | SHAPE OF JUNCTION PORTION | DIAMETER OF REACTION PATH (mm) | LENGTH OF REACTION PATH (m) | REACTION TEMPERATURE (°C) |
|---|---|---|---|---|---|---|---|
| 0.018 | 10 | 0.045 | 20 | CROSS-SHAPE | 5 | 8 | 20 |

~IDL

ID: PR0100 ~PRD

**RELEVANCE DATA**

| FIRST INTERVAL | | SECOND INTERVAL | | 20TH INTERVAL | |
|---|---|---|---|---|---|
| AVERAGE VALUE | 1800 | AVERAGE VALUE | 2000 | AVERAGE VALUE | 380 |
| MAXIMUM VALUE | 2700 | MAXIMUM VALUE | 2200 | MAXIMUM VALUE | 444 |
| MINIMUM VALUE | 45 | MINIMUM VALUE | 1845 | MINIMUM VALUE | 198 |
| MEDIAN VALUE | 1200 | MEDIAN VALUE | 2001 | MEDIAN VALUE | 320 |
| VARIANCE | 50.88 | VARIANCE | 5.96 | VARIANCE | 16.45 |
| SKEWNESS | 0.945 | SKEWNESS | 0.946 | SKEWNESS | 0.152 |
| KURTOSIS | 12.34 | KURTOSIS | 6.32 | KURTOSIS | -18.9 |

~PRD_INT1    ~PRD_INT2    ~PRD_INT20

TEMPORARY MACHINE LEARNING MODEL ~PM

90
SECOND PROCESSING UNIT

POD
**LEARNING OUTPUT DATA**

| MOLECULAR WEIGHT DISPERSION | MOLECULAR WEIGHT |
|---|---|
| 1.5865 | 22000 |

# FIG. 18

ID: PR0100 — PCD

**PRODUCTION CONDITION DATA**

| CONCENTRATION OF FIRST RAW MATERIAL (mol/l) | FLOW VELOCITY OF FIRST RAW MATERIAL (ml/min) | CONCENTRATION OF SECOND RAW MATERIAL (mol/l) | FLOW VELOCITY OF SECOND RAW MATERIAL (ml/min) | SHAPE OF JUNCTION PORTION | DIAMETER OF REACTION PATH (mm) | LENGTH OF REACTION PATH (m) | REACTION TEMPERATURE (°C) |
|---|---|---|---|---|---|---|---|
| 0.018 | 10 | 0.045 | 20 | CROSS-SHAPE | 5 | 8 | 20 |

ID: PR0100 — PRDE

**RELEVANCE DATA**

FIRST INTERVAL

| | |
|---|---|
| AVERAGE VALUE | ☒ |
| MAXIMUM VALUE | 2700 |
| MINIMUM VALUE | 45 |
| MEDIAN VALUE | 1200 |
| VARIANCE | 50.88 |
| SKEWNESS | 0.945 |
| KURTOSIS | 12.34 |

PRD_INT1

SECOND INTERVAL

| | |
|---|---|
| AVERAGE VALUE | 2000 |
| MAXIMUM VALUE | 2200 |
| MINIMUM VALUE | 1845 |
| MEDIAN VALUE | 2001 |
| VARIANCE | 5.96 |
| SKEWNESS | 0.946 |
| KURTOSIS | 6.32 |

PRD_INT2

· · · · · ·

20TH INTERVAL

| | |
|---|---|
| AVERAGE VALUE | 380 |
| MAXIMUM VALUE | 444 |
| MINIMUM VALUE | 198 |
| MEDIAN VALUE | 320 |
| VARIANCE | 16.45 |
| SKEWNESS | 0.152 |
| KURTOSIS | −18.9 |

PRD_INT20

TEMPORARY MACHINE LEARNING MODEL — PM

THIRD PROCESSING UNIT — 91

PODE

**TEMPORARY OUTPUT DATA FOR EXTRACTION**

| MOLECULAR WEIGHT DISPERSION | MOLECULAR WEIGHT |
|---|---|
| 1.6043 | 26000 |

EP 3 995 996 A1

# FIG. 19

92

## CALCULATION UNIT

$$\text{RATE OF CHANGE} = \frac{|\text{DIFFERENCE BETWEEN TEMPORARY OUTPUT DATA AND TEMPORARY OUTPUT DATA FOR EXTRACTION}|}{\text{TEMPORARY OUTPUT DATA}} \quad \text{CALCULATE}$$

⇩

### CONVERT RATE OF CHANGE INTO CONTRIBUTION

| RATE OF CHANGE | CONTRIBUTION |
|---|---|
| EQUAL TO OR LARGER THAN 0 AND SMALLER THAN 0.05 | 0 |
| EQUAL TO OR LARGER THAN 0.05 AND SMALLER THAN 0.1 | 1 |
| EQUAL TO OR LARGER THAN 0.1 AND SMALLER THAN 0.15 | 2 |
| EQUAL TO OR LARGER THAN 0.15 AND SMALLER THAN 0.2 | 3 |
| ⋮ | ⋮ |
| EQUAL TO OR LARGER THAN 0.4 AND SMALLER THAN 0.45 | 8 |
| EQUAL TO OR LARGER THAN 0.45 AND SMALLER THAN 0.5 | 9 |
| EQUAL TO OR LARGER THAN 0.5 | 10 |

100

# FIG. 20

POD

| TEMPORARY OUTPUT DATA | |
|---|---|
| MOLECULAR WEIGHT DISPERSION | MOLECULAR WEIGHT |
| 1.5865 | 22000 |

PODE

| TEMPORARY OUTPUT DATA FOR EXTRACTION | |
|---|---|
| MOLECULAR WEIGHT DISPERSION | MOLECULAR WEIGHT |
| 1.6043 | 26000 |

92

CALCULATION UNIT

$$\text{RATE OF CHANGE IN THE MOLECULAR WEIGHT DISPERSION} = \frac{|1.5865 - 1.6043|}{1.5865} \fallingdotseq 0.01$$

$$\text{RATE OF CHANGE IN THE MOLECULAR WEIGHT} = \frac{|22000 - 26000|}{22000} \fallingdotseq 0.18$$

| RATE OF CHANGE | CONTRIBUTION |
|---|---|
| EQUAL TO OR LARGER THAN 0 AND SMALLER THAN 0.05 | 0 |
| EQUAL TO OR LARGER THAN 0.05 AND SMALLER THAN 0.1 | 1 |
| EQUAL TO OR LARGER THAN 0.1 AND SMALLER THAN 0.15 | 2 |
| EQUAL TO OR LARGER THAN 0.15 AND SMALLER THAN 0.2 | 3 |
| ⋮ | ⋮ |
| EQUAL TO OR LARGER THAN 0.4 AND SMALLER THAN 0.45 | 8 |
| EQUAL TO OR LARGER THAN 0.45 AND SMALLER THAN 0.5 | 9 |
| EQUAL TO OR LARGER THAN 0.5 | 10 |

100

| CONTRIBUTION OBTAINED BY CONVERTING RATE OF CHANGE IN MOLECULAR WEIGHT DISPERSION |
|---|
| 0 |

| CONTRIBUTION OBTAINED BY CONVERTING RATE OF CHANGE IN MOLECULAR WEIGHT |
|---|
| 3 |

# FIG. 21

CI

**CONTRIBUTION INFORMATION**

| FIRST INTERVAL | | |
|---|---|---|
| AVERAGE VALUE | 0 | 3 |
| MAXIMUM VALUE | 8 | 6 |
| MINIMUM VALUE | 2 | 0 |
| MEDIAN VALUE | 1 | 3 |
| VARIANCE | 8 | 8 |
| SKEWNESS | 10 | 10 |
| KURTOSIS | 6 | 8 |

| SECOND INTERVAL | | |
|---|---|---|
| AVERAGE VALUE | 1 | 2 |
| MAXIMUM VALUE | 0 | 2 |
| MINIMUM VALUE | 6 | 5 |
| MEDIAN VALUE | 6 | 7 |
| VARIANCE | 10 | 9 |
| SKEWNESS | 10 | 10 |
| KURTOSIS | 7 | 8 |

. . .          . . .

| 20TH INTERVAL | | |
|---|---|---|
| AVERAGE VALUE | 8 | 8 |
| MAXIMUM VALUE | 10 | 10 |
| MINIMUM VALUE | 6 | 8 |
| MEDIAN VALUE | 1 | 3 |
| VARIANCE | 0 | 2 |
| SKEWNESS | 8 | 6 |
| KURTOSIS | 2 | 0 |

FIG. 22

EP 3 995 996 A1

CONTRIBUTION INFORMATION                                    CI

| FIRST INTERVAL | | |
|---|---|---|
| AVERAGE VALUE | 0 | 3 |
| MAXIMUM VALUE | 8 | 6 |
| MINIMUM VALUE | 2 | 0 |
| MEDIAN VALUE | 1 | 3 |
| VARIANCE | 8 | 8 |
| SKEWNESS | 10 | 10 |
| KURTOSIS | 6 | 8 |

| SECOND INTERVAL | | |
|---|---|---|
| AVERAGE VALUE | 1 | 2 |
| MAXIMUM VALUE | 0 | 2 |
| MINIMUM VALUE | 6 | 5 |
| MEDIAN VALUE | 6 | 7 |
| VARIANCE | 10 | 9 |
| SKEWNESS | 10 | 10 |
| KURTOSIS | 7 | 8 |

| 20TH INTERVAL | | |
|---|---|---|
| AVERAGE VALUE | 8 | 8 |
| MAXIMUM VALUE | 10 | 10 |
| MINIMUM VALUE | 6 | 8 |
| MEDIAN VALUE | 1 | 3 |
| VARIANCE | 0 | 2 |
| SKEWNESS | 8 | 6 |
| KURTOSIS | 2 | 0 |

93

DETERMINATION UNIT

SC

SETTING CONDITION

CONTRIBUTION OBTAINED BY CONVERTING RATE OF CHANGE IN MOLECULAR WEIGHT DISPERSION AND CONTRIBUTION OBTAINED BY CONVERTING RATE OF CHANGE IN MOLECULAR WEIGHT ARE BOTH EQUAL TO OR LARGER THAN 6

HIGH-CONTRIBUTION ITEM INFORMATION

MAXIMUM VALUE, VARIANCE, SKEWNESS, AND KURTOSIS IN FIRST INTERVAL, MEDIAN VALUE, VARIANCE, SKEWNESS, AND KURTOSIS IN SECOND INTERVAL, ···, AND AVERAGE VALUE, MAXIMUM VALUE, MINIMUM VALUE, AND SKEWNESS IN 20TH INTERVAL                    HCII

# FIG. 23

<MAIN LEARNING>

**ID: PR0200** — PCD

IDL

## PRODUCTION CONDITION DATA

| CONCENTRATION OF FIRST RAW MATERIAL (mol/l) | FLOW VELOCITY OF FIRST RAW MATERIAL (ml/min) | CONCENTRATION OF SECOND RAW MATERIAL (mol/l) | FLOW VELOCITY OF SECOND RAW MATERIAL (ml/min) | SHAPE OF JUNCTION PORTION | DIAMETER OF REACTION PATH (mm) | LENGTH OF REACTION PATH (m) | REACTION TEMPERATURE (°C) |
|---|---|---|---|---|---|---|---|
| 0.05 | 8.0 | 0.05 | 8.0 | T-SHAPE | 5 | 8 | 35 |

**ID: PR0200** — PRD

SECOND MACHINE LEARNING MODEL — M2

85

## RELEVANCE DATA

| FIRST INTERVAL | | | SECOND INTERVAL | | | 20TH INTERVAL | |
|---|---|---|---|---|---|---|---|
| AVERAGE VALUE | ✕ | | AVERAGE VALUE | ✕ | | AVERAGE VALUE | 328 |
| MAXIMUM VALUE | 2850 | | MAXIMUM VALUE | ✕ | | MAXIMUM VALUE | 469 |
| MINIMUM VALUE | ✕ | | MINIMUM VALUE | ✕ | | MINIMUM VALUE | 100 |
| MEDIAN VALUE | ✕ | | MEDIAN VALUE | 2005 | | MEDIAN VALUE | ✕ |
| VARIANCE | 55.55 | | VARIANCE | 12.14 | | VARIANCE | ✕ |
| SKEWNESS | 0.875 | | SKEWNESS | 0.982 | | SKEWNESS | 0.246 |
| KURTOSIS | 12.43 | | KURTOSIS | 8.22 | | KURTOSIS | ✕ |

PRD_INT1     PRD_INT2     PRD_INT20

FIRST PROCESSING UNIT

ODL

## LEARNING OUTPUT DATA

| MOLECULAR WEIGHT DISPERSION | MOLECULAR WEIGHT |
|---|---|
| 1.6582 | 25000 |

# FIG. 24

# FIG. 25

**ID: PR0500**  ~PCDF

## PRODUCTION CONDITION DATA FOR PREDICTION

| CONCENTRATION OF FIRST RAW MATERIAL (mol/l) | FLOW VELOCITY OF FIRST RAW MATERIAL (ml/min) | CONCENTRATION OF SECOND RAW MATERIAL (mol/l) | FLOW VELOCITY OF SECOND RAW MATERIAL (ml/min) | SHAPE OF JUNCTION PORTION | DIAMETER OF REACTION PATH (mm) | LENGTH OF REACTION PATH (m) | REACTION TEMPERATURE (°C) |
|---|---|---|---|---|---|---|---|
| 0.35 | 15 | 0.35 | 15 | T-SHAPE | 5 | 10 | 20 |

**ID: PR0500**  ~PRDF

## RELEVANCE DATA FOR PREDICTION

| FIRST INTERVAL | |
|---|---|
| AVERAGE VALUE | ✕ |
| MAXIMUM VALUE | 2400 |
| MINIMUM VALUE | ✕ |
| MEDIAN VALUE | ✕ |
| VARIANCE | 92.46 |
| SKEWNESS | 0.962 |
| KURTOSIS | 18.32 |

~PRD_INT1

| SECOND INTERVAL | |
|---|---|
| AVERAGE VALUE | ✕ |
| MAXIMUM VALUE | ✕ |
| MINIMUM VALUE | ✕ |
| MEDIAN VALUE | 2002 |
| VARIANCE | 14.45 |
| SKEWNESS | 0.954 |
| KURTOSIS | 8.93 |

~PRD_INT2

· · · · · · ·

| 20TH INTERVAL | |
|---|---|
| AVERAGE VALUE | 425 |
| MAXIMUM VALUE | 501 |
| MINIMUM VALUE | 126 |
| MEDIAN VALUE | ✕ |
| VARIANCE | ✕ |
| SKEWNESS | 0.168 |
| KURTOSIS | ✕ |

~PRD_INT20

LEARNED MODEL ~TM

117

FOURTH PROCESSING UNIT

QFD

## QUALITY PREDICTION DATA

| MOLECULAR WEIGHT DISPERSION | MOLECULAR WEIGHT |
|---|---|
| 1.5555 | 23500 |

# FIG. 26

QUALITY PREDICTION APPLICATION
– DISPLAYING OF QUALITY PREDICTION DATA ~120

QUALITY PREDICTION DATA
OF PRODUCT IS DISPLAYED.

QUALITY PREDICTION DATA ~QFD

| MOLECULAR WEIGHT DISPERSION | MOLECULAR WEIGHT |
|---|---|
| 1.5555 | 23500 |

OK ~121

# FIG. 27

START

ST100 — TEMPORARY LEARNING

ST200 — EXTRACTION OF HIGH-CONTRIBUTION ITEMS

ST300 — MAIN LEARNING

END

FIRST MACHINE LEARNING MODEL ~M1

TEMPORARY MACHINE LEARNING MODEL ~PM

TEMPORARY MACHINE LEARNING MODEL ~PM

SECOND MACHINE LEARNING MODEL ~M2

LEARNED MODEL ~TM

## FIG. 28

```
        ┌─────────────────────────┐
        │      START OF           │
        │   TEMPORARY LEARNING    │
        └─────────────────────────┘
                    │
ST1001 ┌────────────────────────────────────────┐
       │    READ(ACQUIRE) LEARNING INPUT DATA    │
       └────────────────────────────────────────┘
                    │
ST1002 ┌────────────────────────────────────────┐
       │ OUTPUT LEARNING OUTPUT DATA BY INPUTTING LEARNING │
       │ INPUT DATA TO FIRST MACHINE LEARNING MODEL        │
       └────────────────────────────────────────┘
                    │
ST1003 ┌────────────────────────────────────────┐      ST1005 ┌──────────────────────────────────┐
       │ EVALUATE ACCURACY OF PREDICTION OF QUALITY OF │       │ UPDATE FIRST MACHINE LEARNING MODEL │
       │ PRODUCT BY FIRST MACHINE LEARNING MODEL BY    │       └──────────────────────────────────┘
       │ COMPARING LEARNING OUTPUT DATA AND QUALITY DATA │
       └────────────────────────────────────────┘
                    │
ST1004  < DOES ACCURACY REACH PRESET LEVEL? >  NO
                    │ YES
ST1006 ┌────────────────────────────────────────┐
       │ OUTPUT TEMPORARY MACHINE LEARNING MODEL │
       └────────────────────────────────────────┘
                    │
              ┌───────────┐
              │    END    │
              └───────────┘
```

EP 3 995 996 A1

# FIG. 29

START OF EXTRACTION OF
HIGH-CONTRIBUTION ITEMS

ST2001
OUTPUT TEMPORARY OUTPUT DATA BY INPUTTING LEARNING
INPUT DATA TO TEMPORARY MACHINE LEARNING MODEL

ST2002
OUTPUT TEMPORARY OUTPUT DATA FOR EXTRACTION BY
INPUTTING PRODUCTION CONDITION DATA AND RELEVANCE DATA
FOR EXTRACTION TO TEMPORARY MACHINE LEARNING MODEL

ST2003
CALCULATE CONTRIBUTION BASED ON TEMPORARY OUTPUT
DATA AND TEMPORARY OUTPUT DATA FOR EXTRACTION

ST2004
ARE CONTRIBUTIONS OF ALL ITEMS OF
RELEVANCE DATA CALCULATED?          NO

YES

ST2005
DETERMINE WHETHER OR NOT CONTRIBUTION OF EACH OF
ITEMS OF RELEVANCE DATA SATISFIES PRESET CONDITION

ST2006
OUTPUT DETERMINATION RESULT AS
HIGH-CONTRIBUTION ITEM INFORMATION

END

EP 3 995 996 A1

## FIG. 30

```
        ( START OF MAIN LEARNING )
                    │
ST3001 ┐            ▼
┌──────────────────────────────────────────────┐
│        READ(ACQUIRE) LEARNING INPUT DATA       │
└──────────────────────────────────────────────┘
                    │
ST3002 ┐            ▼
┌──────────────────────────────────────────────┐
│  OUTPUT LEARNING OUTPUT DATA BY SELECTIVELY    │
│     INPUTTING PRODUCTION CONDITION DATA AND    │
│  RELEVANCE DATA OF HIGH-CONTRIBUTION ITEM TO   │
│         SECOND MACHINE LEARNING MODEL          │
└──────────────────────────────────────────────┘
                    │
ST3003 ┐            ▼                               ST3005 ┐
┌──────────────────────────────────────────────┐   ┌──────────────────────────────────────┐
│   EVALUATE ACCURACY OF PREDICTION OF QUALITY   │   │ UPDATE SECOND MACHINE LEARNING MODEL   │
│    OF PRODUCT BY SECOND MACHINE LEARNING       │   └──────────────────────────────────────┘
│   MODEL BY COMPARING LEARNING OUTPUT DATA      │
│              AND QUALITY DATA                   │
└──────────────────────────────────────────────┘
                    │
ST3004 ┐            ▼
< DOES ACCURACY REACH PRESET LEVEL? >──── NO
                    │
ST3006 ┐          YES
┌──────────────────────────────────────────────┐
│             OUTPUT LEARNED MODEL               │
└──────────────────────────────────────────────┘
                    │
                    ▼
                ( END )
```

# FIG. 31

START

ST500 — READ(ACQUIRE) LEARNED MODEL

ST600 — READ(ACQUIRE) PRODUCTION CONDITION DATA FOR PREDICTION AND RELEVANCE DATA FOR PREDICTION

ST700 — OUTPUT QUALITY PREDICTION DATA BY INPUTTING PRODUCTION CONDITION DATA FOR PREDICTION AND RELEVANCE DATA FOR PREDICTION TO LEARNED MODEL

ST800 — OUTPUT QUALITY PREDICTION DATA

END

# FIG. 32

125

| | DETERMINATION COEFFICIENT OF MOLECULAR WEIGHT DISPERSION | DETERMINATION COEFFICIENT OF MOLECULAR WEIGHT |
|---|---|---|
| COMPARATIVE EXAMPLE 1 | 0.6 | 0.65 |
| COMPARATIVE EXAMPLE 2 | 0.82 | 0.81 |
| EXAMPLE | 0.98 | 0.95 |

COMPARATIVE EXAMPLE 1: LEARNING INPUT DATA = ONLY PRODUCTION CONDITION DATA

COMPARATIVE EXAMPLE 2: LEARNING INPUT DATA = PRODUCTION CONDITION DATA + RELEVANCE DATA

EXAMPLE: LEARNING INPUT DATA = PRODUCTION CONDITION DATA + RELEVANCE DATA OF HIGH-CONTRIBUTION ITEM

## FIG. 33

## FIG. 34

## FIG. 35

EP 3 995 996 A1

# FIG. 36

AE

AUTOENCODER

(IIMD)PD

PHYSICAL-PROPERTY DATA
(INPUT IMAGE DATA)

RELEVANCE DATA

PRD

140

FIRST DERIVATION UNIT

## FIG. 37

EP 3 995 996 A1

# FIG. 38

EP 3 995 996 A1

DI — 

Ip

Is

| a | b | c |
| d | e | f |
| g | h | i |

Is

x

y

→ CONVOLUTION PROCESSING →

F —

| r | s | t |
| u | v | w |
| x | y | z |

DIc —

Icp

| k |

x

y

# FIG. 39

NUMBER OF CHANNELS

DIc
DIc
CMP
DIc
DIc

HEIGHT

WIDTH

# FIG. 40

INT1 INT2 INT3 ... SPIMD ... INT20

SP

INTENSITY

WAVE NUMBER

SPIMD_INT1

SPIMD_INT3

SPIMD_INT20

AE_INT1 AE_INT2 AE_INT3 ... AE_INT20

## FIG. 41

LEARNING INPUT IMAGE DATA
(IMAGE DATA OF SPECTRUM OF
PRODUCT OF WHICH QUALITY IS
HIGHER THAN PRESET LEVEL)

IIMDL

OIMDL

LEARNING OUTPUT IMAGE DATA

INT1

SP

SPIMD_INT1

SP

AE_INT1

AUTOENCODER

PERFORM LEARNING SUCH THAT LEARNING INPUT
IMAGE DATA AND LEARNING OUTPUT IMAGE DATA
MATCH WITH EACH OTHER

EP 3 995 996 A1

## FIG. 42

FIG. 43

DD_INT1

DD_INT2 — DIFFERENCE DATA (SECOND INTERVAL)

DD_INT20 — DIFFERENCE DATA (20TH INTERVAL)

**DIFFERENCE DATA (FIRST INTERVAL)**

| WAVE NUMBER | DIFFERENCE IN INTENSITY |
|---|---|
| 650 | 12 |
| 652 | 8 |
| 655 | 6 |
| ⋯ | ⋯ |

**DIFFERENCE DATA (SECOND INTERVAL)**

| WAVE NUMBER | DIFFERENCE IN INTENSITY |
|---|---|
| 720 | −11 |
| 722 | −15 |
| 725 | −13 |
| ⋯ | ⋯ |

**DIFFERENCE DATA (20TH INTERVAL)**

| WAVE NUMBER | DIFFERENCE IN INTENSITY |
|---|---|
| 1930 | 1 |
| 1932 | 0 |
| 1935 | −1 |
| ⋯ | ⋯ |

140 — FIRST DERIVATION UNIT

PRD_INT1

PRD_INT2 — RELEVANCE DATA (SECOND INTERVAL)

PRD_INT20 — RELEVANCE DATA (20TH INTERVAL)

**RELEVANCE DATA (FIRST INTERVAL)**

| AVERAGE VALUE | 6.5 |
|---|---|
| SUM | 256 |

**RELEVANCE DATA (SECOND INTERVAL)**

| AVERAGE VALUE | −4.85 |
|---|---|
| SUM | −194 |

**RELEVANCE DATA (20TH INTERVAL)**

| AVERAGE VALUE | −0.8 |
|---|---|
| SUM | −32.2 |

## FIG. 44

INT1

IIMD

SP

INPUT IMAGE DATA

SPIMD_INT1

AE_INT1

AUTOENCODER

155

ENCODER NETWORK

150

FIRST DERIVATION UNIT

PRD_INT1

RELEVANCE DATA

IMAGE FEATURE MAP

CMP

DIc — Ch1

DIc — Ch2

DIc — Ch3

DIc — Ch4

AVERAGE VALUE OF PIXEL VALUE OF OUTPUT DATA OF CHANNEL 1

AVERAGE VALUE OF PIXEL VALUE OF OUTPUT DATA OF CHANNEL 1

AVERAGE VALUE OF PIXEL VALUE OF OUTPUT DATA OF CHANNEL 1

AVERAGE VALUE OF PIXEL VALUE OF OUTPUT DATA OF CHANNEL 1

EP 3 995 996 A1

<div style="text-align:center">**INTERNATIONAL SEARCH REPORT**</div>

| | International application No. |
|---|---|
| | PCT/JP2020/020026 |

A. CLASSIFICATION OF SUBJECT MATTER
Int. Cl. G06N3/04(2006.01)i, G06N20/00(2019.01)i
FI: G06N20/00, G06N3/04

According to International Patent Classification (IPC) or to both national classification and IPC

B. FIELDS SEARCHED

Minimum documentation searched (classification system followed by classification symbols)
Int. Cl. G06N3/00-99/00

Documentation searched other than minimum documentation to the extent that such documents are included in the fields searched
Published examined utility model applications of Japan     1922-1996
Published unexamined utility model applications of Japan   1971-2020
Registered utility model specifications of Japan           1996-2020
Published registered utility model applications of Japan   1994-2020

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | 長田典子、外2名，ニューラルネットの感度特性を用いた要因特定法と真珠色彩識別への応用，電気学会論文誌 C, 20 April 1996, vol. 116-C, no. 5, pp. 556-562, 特に，「2.ニューラルネットワークの感度特性を用いた要因特定法」-「4.要因特定法のスペクトルへの応用」 (NATAGA, Noriko et al., Factors Identification Using Sensitivity of Layered Neural Networks and Its Application to Pearl Color Evaluation. IEEJ Transactions C. In particular, 2. Factor Identification Method using Sensitivity of Layered Neural Networks, 4. Application to the Factor Identification Method.) | 1-14 |
| Y | JP 6-304724 A (NIPPON STEEL CORP.) 01 November 1994, paragraph [0042] | 1-14 |

☒ Further documents are listed in the continuation of Box C.  ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 06.07.2020 | 21.07.2020 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2020/020026 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 2016-109495 A (TAKANO CO., LTD.) 20 June 2016, paragraphs [0052], [0061]-[0066] | 1-14 |
| Y | WO 2019/073666 A1 (NIKON CORP.) 18 April 2019, paragraph [0065], fig. 8 | 4, 11 |
| Y | JP 2016-91359 A (RICOH CO., LTD.) 23 May 2016, paragraphs [0047]-[0053], [0140]-[0143], [0153]-[0172], fig. 4, 21 | 7-8, 10-11 |
| Y | JP 2018-5773 A (RICOH CO., LTD.) 11 January 2018, paragraphs [0020], [0027]-[0032] | 7, 9-11 |
| Y | JP 2018-116054 A (KAGAWA UNIVERSITY) 26 July 2018, paragraphs [0110]-[0122] | 10-11 |

Form PCT/ISA/210 (continuation of second sheet) (January 2015)

INTERNATIONAL SEARCH REPORT

Information on patent family members

International application No.

PCT/JP2020/020026

| Patent Documents referred to in the Report | Publication Date | Patent Family | Publication Date |
|---|---|---|---|
| JP 6-304724 A | 01.11.1994 | (Family: none) | |
| JP 2016-109495 A | 20.06.2016 | (Family: none) | |
| WO 2019/073666 A1 | 18.04.2019 | (Family: none) | |
| JP 2016-91359 A | 23.05.2016 | (Family: none) | |
| JP 2018-5773 A | 11.01.2018 | (Family: none) | |
| JP 2018-116054 A | 26.07.2018 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2018018354 A

**Non-patent literature cited in the description**

• Regularization Paths for Generalized Linear Models via Coordinate Descent. *Journal of statistical software,* 2010, vol. 33 (1